(19) Europäisches Patentamt / European Patent Office / Office européen des brevets

(11) **EP 3 450 461 A1**

(12) **EUROPEAN PATENT APPLICATION**
published in accordance with Art. 153(4) EPC

(43) Date of publication:
06.03.2019 Bulletin 2019/10

(51) Int Cl.:
*C07K 19/00* (2006.01)     *C07K 14/36* (2006.01)
*C12N 15/62* (2006.01)     *C07K 16/12* (2006.01)
*A61K 39/02* (2006.01)     *A61P 31/04* (2006.01)
*G01N 33/53* (2006.01)

(21) Application number: 16785935.4

(22) Date of filing: 28.04.2016

(86) International application number:
PCT/CN2016/080469

(87) International publication number:
WO 2016/173504 (03.11.2016 Gazette 2016/44)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(71) Applicant: **Golden Scorpio International Co., Ltd.
Taichung City, Taiwan 40460 (TW)**

(72) Inventors:
• **YANG, Ying-Chen
Taipei City 10556 (TW)**
• **KUO, Tsun-Yung
I-Lan County
Taiwan 26341 (TW)**

(74) Representative: **Wittmann, Günther
Patentanwaltskanzlei Wittmann
Frans-Hals-Straße 31
81479 München (DE)**

(54) **ACTINOBACILLUS PLEUROPNEUMONIAE RECOMBINANT TOXIN PROTEIN AND APPLICATION THEREOF**

(57) The present invention provides an *Actinobacillus pleuropneumoniae* recombinant toxin protein, comprising at least one antigenic determinant of an *Actinobacillus pleuropneumoniae* toxin protein; and when there is a plurality of antigenic determinants, connexons can exist among the antigenic determinants. A recombinant protein may comprise at least one amino acid sequence of a complement fragmentation C3d, and connexons can exist between antigenic determinants and the amino acid sequences of the C3d. The present invention also provides a nucleotide sequence for encoding a protein, and an immune composition containing the protein. (Provided by the WIPO)

SEQUENCE LISTING

<110>  Golden Scorpio International Co., Ltd.

<120>  Actinobacillus pleuropneumoniae Recombinant Toxin Protein And
       Application Thereof

<130>  P18-0174

<140>  PCT/CN2016/080469
<141>  2016-04-28

<160>  117

<170>  PatentIn version 3.5

<210>  1
<211>  1022
<212>  PRT
<213>  Actinobacillus pleuropneumoniae

<400>  1

Met Ala Asn Ser Gln Leu Asp Arg Val Lys Gly Leu Ile Asp Ser Leu
1               5                   10                  15


Asn Gln His Thr Lys Ser Ala Ala Lys Ser Gly Ala Gly Ala Leu Lys
            20                  25                  30


Asn Gly Leu Gly Gln Val Lys Gln Ala Gly Gln Lys Leu Ile Leu Tyr
        35                  40                  45


Ile Pro Lys Asp Tyr Gln Ala Ser Thr Gly Ser Ser Leu Asn Asp Leu
    50                  55                  60


Val Lys Ala Ala Glu Ala Leu Gly Ile Glu Val His Arg Ser Glu Lys
65                  70                  75                  80


Asn Gly Thr Ala Leu Ala Lys Glu Leu Phe Gly Thr Thr Glu Lys Leu
                85                  90                  95


Leu Gly Phe Ser Glu Arg Gly Ile Ala Leu Phe Ala Pro Gln Phe Asp
            100                 105                 110


Lys Leu Leu Asn Lys Asn Gln Lys Leu Ser Lys Ser Leu Gly Gly Ser
        115                 120                 125


Ser Glu Ala Leu Gly Gln Arg Leu Asn Lys Thr Gln Thr Ala Leu Ser
        130                 135                 140


Ala Leu Gln Ser Phe Leu Gly Thr Ala Ile Ala Gly Met Asp Leu Asp
145                 150                 155                 160

```
Ser Leu Leu Arg Arg Arg Arg Asn Gly Glu Asp Val Ser Gly Ser Glu
            165             170             175

Leu Ala Lys Ala Gly Val Asp Leu Ala Ala Gln Leu Val Asp Asn Ile
            180             185             190

Ala Ser Ala Thr Gly Thr Val Asp Ala Phe Ala Glu Gln Leu Gly Lys
            195             200             205

Leu Gly Asn Ala Leu Ser Asn Thr Arg Leu Ser Gly Leu Ala Ser Lys
    210             215             220

Leu Asn Asn Leu Pro Asp Leu Ser Leu Ala Gly Pro Gly Phe Asp Ala
225             230             235             240

Val Ser Gly Ile Leu Ser Val Val Ser Ala Ser Phe Ile Leu Ser Asn
            245             250             255

Lys Asp Ala Asp Ala Gly Thr Lys Ala Ala Ala Gly Ile Glu Ile Ser
            260             265             270

Thr Lys Ile Leu Gly Asn Ile Gly Lys Ala Val Ser Gln Tyr Ile Ile
            275             280             285

Ala Gln Arg Val Ala Ala Gly Leu Ser Thr Thr Ala Ala Thr Arg Trp
    290             295             300

Phe Asn Arg Ser Val Val Ala Leu Ala Ile Ser Pro Leu Ser Phe Leu
305             310             315             320

Asn Val Ala Asp Lys Phe Glu Arg Ala Lys Gln Leu Glu Gln Tyr Ser
            325             330             335

Glu Arg Phe Lys Lys Phe Gly Tyr Glu Gly Asp Ser Leu Leu Ala Ser
            340             345             350

Phe Tyr Arg Glu Thr Gly Ala Ile Glu Ala Ala Leu Thr Thr Ile Asn
            355             360             365

Ser Val Leu Ser Ala Ala Pro Ala Gly Val Gly Ala Ala Ala Thr Gly
    370             375             380

Ser Leu Val Gly Ala Pro Val Ala Ala Leu Val Ser Ala Ile Thr Gly
385             390             395             400

Ile Ile Ser Gly Ile Leu Asp Ala Ser Lys Gln Ala Ile Phe Glu Arg
            405             410             415
```

```
Val Ala Thr Lys Leu Ala Asn Lys Ile Asp Glu Trp Glu Lys Lys His
        420             425             430

Gly Lys Asn Tyr Phe Glu Asn Gly Tyr Asp Ala Arg His Ser Ala Phe
        435             440             445

Leu Glu Asp Thr Phe Glu Leu Leu Ser Gln Tyr Asn Lys Glu Tyr Ser
    450             455             460

Val Glu Arg Val Val Ala Ile Thr Gln Gln Arg Trp Asp Val Asn Ile
465             470             475             480

Gly Glu Leu Ala Gly Ile Thr Arg Lys Gly Ala Asp Ala Lys Ser Gly
                485             490             495

Lys Ala Tyr Val Asp Phe Phe Glu Glu Gly Lys Leu Leu Glu Lys Asp
        500             505             510

Pro Asp Arg Phe Asp Lys Lys Val Phe Asp Pro Leu Glu Gly Lys Ile
        515             520             525

Asp Leu Ser Ser Ile Asn Lys Thr Thr Leu Leu Lys Phe Ile Thr Pro
    530             535             540

Ala Phe Thr Ala Gly Glu Glu Ile Arg Glu Arg Lys Gln Thr Gly Lys
545             550             555             560

Tyr Glu Tyr Met Thr Glu Leu Phe Val Lys Gly Lys Glu Lys Trp Val
                565             570             575

Val Thr Gly Val Gln Ser His Asn Ala Ile Tyr Asp Tyr Thr Asn Leu
            580             585             590

Ile Gln Leu Ala Ile Asp Lys Lys Gly Glu Lys Arg Gln Val Thr Ile
        595             600             605

Glu Ser His Leu Gly Glu Lys Asn Asp Arg Ile Tyr Leu Ser Ser Gly
    610             615             620

Ser Ser Ile Glu Tyr Ala Gly Asn Gly His Asp Val Ala Tyr Tyr Asp
625             630             635             640

Lys Thr Asp Thr Gly Tyr Leu Thr Phe Asp Gly Gln Ser Ala Gln Lys
                645             650             655

Ala Gly Glu Tyr Ile Val Thr Lys Glu Leu Lys Ala Asp Val Lys Val
        660             665             670
```

18

```
Leu Lys Glu Val Val Lys Thr Gln Asp Ile Ser Val Gly Lys Arg Ser
        675                 680             685

Glu Lys Leu Glu Tyr Arg Asp Tyr Glu Leu Ser Pro Phe Glu Leu Gly
        690                 695             700

Asn Gly Ile Arg Ala Lys Asp Glu Leu His Ser Val Glu Glu Ile Ile
705             710             715                 720

Gly Ser Asn Arg Lys Asp Lys Phe Phe Gly Ser Arg Phe Thr Asp Ile
                725             730                 735

Phe His Gly Ala Lys Gly Asp Asp Glu Ile Tyr Gly Asn Asp Gly His
            740             745             750

Asp Ile Leu Tyr Gly Asp Asp Gly Asn Asp Val Ile His Gly Gly Asp
        755             760             765

Gly Asn Asp His Leu Val Gly Gly Asn Gly Asn Asp Arg Leu Ile Gly
    770             775             780

Gly Lys Gly Asn Asn Phe Leu Asn Gly Gly Asp Gly Asp Asp Glu Leu
785             790             795                 800

Gln Val Phe Glu Cys Gln Tyr Asn Val Leu Leu Gly Gly Ala Gly Asn
                805             810             815

Asp Ile Leu Tyr Gly Ser Asp Gly Thr Asn Leu Phe Asp Gly Gly Val
        820             825             830

Gly Asn Asp Lys Ile Tyr Gly Gly Leu Gly Lys Asp Ile Tyr Arg Tyr
        835             840             845

Ser Lys Glu Tyr Gly Arg His Ile Ile Ile Glu Lys Gly Gly Asp Asp
    850             855             860

Asp Thr Leu Leu Leu Ser Asp Leu Ser Phe Lys Asp Val Gly Phe Ile
865             870             875                 880

Arg Ile Gly Asp Asp Leu Leu Val Asn Lys Arg Ile Gly Gly Thr Leu
            885             890             895

Tyr Tyr His Glu Asp Tyr Asn Gly Asn Ala Leu Thr Ile Lys Asp Trp
        900             905             910

Phe Lys Glu Gly Lys Glu Gly Gln Asn Asn Lys Val Glu Lys Ile Val
```

```
                915                    920                        925


        Asp Lys Asp Gly Ala Tyr Val Leu Ser Gln Tyr Leu Thr Glu Leu Thr
            930                    935                    940


        Ala Pro Gly Arg Gly Ile Asn Tyr Phe Asn Gly Leu Glu Glu Lys Leu
        945                    950                    955                    960


        Tyr Tyr Gly Glu Gly Tyr Asn Ala Leu Pro Gln Leu Arg Lys Asp Ile
                    965                    970                    975


        Glu Gln Ile Ile Ser Ser Thr Gly Ala Leu Thr Gly Glu His Gly Gln
                    980                    985                    990


        Val Leu Val Gly Ala Gly Gly Pro  Leu Ala Tyr Ser Asn  Ser Pro Asn
                995                    1000                    1005


        Ser Ile  Pro Asn Ala Phe Ser  Asn Tyr Leu Thr Gln  Ser Ala
            1010                    1015                    1020


        <210>  2
        <211>  16
        <212>  PRT
        <213>  Actinobacillus pleuropneumoniae

        <400>  2

        Glu Leu Ala Gly Ile Thr Arg Lys Gly Ala Asp Ala Lys Ser Gly Lys
        1                  5                  10                  15


        <210>  3
        <211>  8
        <212>  PRT
        <213>  Actinobacillus pleuropneumoniae

        <400>  3

        Pro Ala Gly Val Gly Ala Ala Ala
        1                  5


        <210>  4
        <211>  24
        <212>  PRT
        <213>  Actinobacillus pleuropneumoniae

        <400>  4

        Asp Ile Leu Tyr Gly Ser Asp Gly Thr Asn Leu Phe Asp Gly Gly Val
        1                  5                  10                  15


        Gly Asn Asp Lys Ile Tyr Gly Gly
                    20
```

<210> 5
<211> 26
<212> PRT
<213> Actinobacillus pleuropneumoniae

<400> 5

Glu His Gly Gln Val Leu Val Gly Ala Gly Gly Pro Leu Ala Tyr Ser
1               5                   10                  15

Asn Ser Pro Asn Ser Ile Pro Asn Ala Phe
            20                  25

<210> 6
<211> 97
<212> PRT
<213> Actinobacillus pleuropneumoniae

<400> 6

Arg Ala Lys Asp Glu Leu His Ser Val Glu Glu Ile Ile Gly Ser Asn
1               5                   10                  15

Arg Lys Asp Lys Phe Phe Gly Ser Arg Phe Thr Asp Ile Phe His Gly
            20                  25                  30

Ala Lys Gly Asp Asp Glu Ile Tyr Gly Asn Asp Gly His Asp Ile Leu
            35                  40                  45

Tyr Gly Asp Asp Gly Asn Asp Val Ile His Gly Gly Asp Gly Asn Asp
    50                  55                  60

His Leu Val Gly Gly Asn Gly Asn Asp Arg Leu Ile Gly Gly Lys Gly
65                  70                  75                  80

Asn Asn Phe Leu Asn Gly Gly Asp Gly Asp Asp Glu Leu Gln Val Phe
                85                  90                  95

Glu

<210> 7
<211> 410
<212> PRT
<213> Artificial Sequence

<220>
<223> Recombinant ApxI toxin

<400> 7

Glu Leu Ala Gly Ile Thr Arg Lys Gly Ala Asp Ala Lys Ser Gly Lys
1               5                   10                  15

Gly Ser Thr Ala Pro Ala Gly Val Gly Ala Ala Ala Gly Ser Thr Ala
                20              25              30

Gly Ser Thr Ala Asp Ile Leu Tyr Gly Ser Asp Gly Thr Asn Leu Phe
                35              40              45

Asp Gly Gly Val Gly Asn Asp Lys Ile Tyr Gly Gly Gly Ser Thr Ala
        50              55              60

Glu His Gly Gln Val Leu Val Gly Ala Gly Gly Pro Leu Ala Tyr Ser
65              70              75              80

Asn Ser Pro Asn Ser Ile Pro Asn Ala Phe Gly Ser Thr Ala Arg Ala
                85              90              95

Lys Asp Glu Leu His Ser Val Glu Glu Ile Ile Gly Ser Asn Arg Lys
                100             105             110

Asp Lys Phe Phe Gly Ser Arg Phe Thr Asp Ile Phe His Gly Ala Lys
        115             120             125

Gly Asp Asp Glu Ile Tyr Gly Asn Asp Gly His Asp Ile Leu Tyr Gly
        130             135             140

Asp Asp Gly Asn Asp Val Ile His Gly Gly Asp Gly Asn Asp His Leu
145             150             155             160

Val Gly Gly Asn Gly Asn Asp Arg Leu Ile Gly Gly Lys Gly Asn Asn
                165             170             175

Phe Leu Asn Gly Gly Asp Gly Asp Asp Glu Leu Leu Gln Val Phe Glu
                180             185             190

Gly Ser Thr Ala Gly Ser Thr Ala Phe Leu Asn Lys Leu Leu Ser Thr
                195             200             205

Ala Lys Glu Arg Asn Arg Trp Glu Glu Pro Gly Gln Lys Leu Tyr Asn
        210             215             220

Val Glu Ala Thr Ser Tyr Ala Gly Ser Thr Ala Phe Leu Asn Lys Leu
225             230             235             240

Leu Ser Thr Ala Lys Glu Arg Asn Arg Trp Glu Glu Pro Gly Gln Lys
                245             250             255

Leu Tyr Asn Val Glu Ala Thr Ser Tyr Ala Gly Ser Thr Ala Phe Leu

260     265     270

Asn Lys Leu Leu Ser Thr Ala Lys Glu Arg Asn Arg Trp Glu Glu Pro
  275     280     285

Gly Gln Lys Leu Tyr Asn Val Glu Ala Thr Ser Tyr Ala Gly Ser Thr
 290     295     300

Ala Phe Leu Asn Lys Leu Leu Ser Thr Ala Lys Glu Arg Asn Arg Trp
305     310     315     320

Glu Glu Pro Gly Gln Lys Leu Tyr Asn Val Glu Ala Thr Ser Tyr Ala
   325     330     335

Gly Ser Thr Ala Phe Leu Asn Lys Leu Leu Ser Thr Ala Lys Glu Arg
  340     345     350

Asn Arg Trp Glu Glu Pro Gly Gln Lys Leu Tyr Asn Val Glu Ala Thr
  355     360     365

Ser Tyr Ala Gly Ser Thr Ala Phe Leu Asn Lys Leu Leu Ser Thr Ala
 370     375     380

Lys Glu Arg Asn Arg Trp Glu Glu Pro Gly Gln Lys Leu Tyr Asn Val
385     390     395     400

Glu Ala Thr Ser Tyr Ala Gly Ser Thr Ala
    405     410

<210> 8
<211> 412
<212> PRT
<213> Artificial Sequence

<220>
<223> Recombinant ApxI toxin with a HindIII site

<400> 8

Glu Leu Ala Gly Ile Thr Arg Lys Gly Ala Asp Ala Lys Ser Gly Lys
1     5     10     15

Gly Ser Thr Ala Pro Ala Gly Val Gly Ala Ala Ala Gly Ser Thr Ala
  20     25     30

Gly Ser Thr Ala Asp Ile Leu Tyr Gly Ser Asp Gly Thr Asn Leu Phe
  35     40     45

Asp Gly Gly Val Gly Asn Asp Lys Ile Tyr Gly Gly Gly Ser Thr Ala
 50     55     60

```
Glu His Gly Gln Val Leu Val Gly Ala Gly Gly Pro Leu Ala Tyr Ser
65              70              75                   80


Asn Ser Pro Asn Ser Ile Pro Asn Ala Phe Gly Ser Thr Ala Arg Ala
                85              90                   95


Lys Asp Glu Leu His Ser Val Glu Glu Ile Ile Gly Ser Asn Arg Lys
            100             105             110


Asp Lys Phe Phe Gly Ser Arg Phe Thr Asp Ile Phe His Gly Ala Lys
        115             120             125


Gly Asp Asp Glu Ile Tyr Gly Asn Asp Gly His Asp Ile Leu Tyr Gly
    130             135             140


Asp Asp Gly Asn Asp Val Ile His Gly Gly Asp Gly Asn Asp His Leu
145             150             155             160


Val Gly Gly Asn Gly Asn Asp Arg Leu Ile Gly Gly Lys Gly Asn Asn
            165             170             175


Phe Leu Asn Gly Gly Asp Gly Asp Asp Glu Leu Leu Gln Val Phe Glu
        180             185             190


Gly Ser Thr Ala Lys Leu Gly Ser Thr Ala Phe Leu Asn Lys Leu Leu
    195             200             205


Ser Thr Ala Lys Glu Arg Asn Arg Trp Glu Glu Pro Gly Gln Lys Leu
210             215             220


Tyr Asn Val Glu Ala Thr Ser Tyr Ala Gly Ser Thr Ala Phe Leu Asn
225             230             235             240


Lys Leu Leu Ser Thr Ala Lys Glu Arg Asn Arg Trp Glu Glu Pro Gly
            245             250             255


Gln Lys Leu Tyr Asn Val Glu Ala Thr Ser Tyr Ala Gly Ser Thr Ala
        260             265             270


Phe Leu Asn Lys Leu Leu Ser Thr Ala Lys Glu Arg Asn Arg Trp Glu
        275             280             285


Glu Pro Gly Gln Lys Leu Tyr Asn Val Glu Ala Thr Ser Tyr Ala Gly
    290             295             300


Ser Thr Ala Phe Leu Asn Lys Leu Leu Ser Thr Ala Lys Glu Arg Asn
```

```
              305                   310                   315                   320


    Arg Trp Glu Glu Pro Gly Gln Lys Leu Tyr Asn Val Glu Ala Thr Ser
                    325                   330                   335


    Tyr Ala Gly Ser Thr Ala Phe Leu Asn Lys Leu Leu Ser Thr Ala Lys
                340                   345                   350


    Glu Arg Asn Arg Trp Glu Glu Pro Gly Gln Lys Leu Tyr Asn Val Glu
            355                   360                   365


    Ala Thr Ser Tyr Ala Gly Ser Thr Ala Phe Leu Asn Lys Leu Leu Ser
        370                   375                   380


    Thr Ala Lys Glu Arg Asn Arg Trp Glu Glu Pro Gly Gln Lys Leu Tyr
    385                   390                   395                   400


    Asn Val Glu Ala Thr Ser Tyr Ala Gly Ser Thr Ala
                    405                   410
```

<210> 9
<211> 956
<212> PRT
<213> Actinobacillus pleuropneumoniae

<400> 9

```
    Met Ser Lys Ile Thr Leu Ser Ser Leu Lys Ser Ser Leu Gln Gln Gly
    1               5                   10                  15


    Leu Lys Asn Gly Lys Asn Lys Leu Asn Gln Ala Gly Thr Thr Leu Lys
                20                  25                  30


    Asn Gly Leu Thr Gln Thr Gly His Ser Leu Gln Asn Gly Ala Lys Lys
                35                  40                  45


    Leu Ile Leu Tyr Ile Pro Gln Gly Tyr Asp Ser Gly Gln Gly Asn Gly
        50                  55                  60


    Val Gln Asp Leu Val Lys Ala Ala Asn Asp Leu Gly Ile Glu Val Trp
    65                  70                  75                  80


    Arg Glu Glu Arg Ser Asn Leu Asp Ile Ala Lys Thr Ser Phe Asp Thr
                85                  90                  95


    Thr Gln Lys Ile Leu Gly Phe Thr Asp Arg Gly Ile Val Leu Phe Ala
                100                 105                 110


    Pro Gln Leu Asp Asn Leu Leu Lys Lys Asn Pro Lys Ile Gly Asn Thr
```

|     | 115 |     |     | 120 |     |     | 125 |

Leu Gly Ser Ala Ser Ser Ile Ser Gln Asn Ile Gly Lys Ala Asn Thr
    130             135             140

Val Leu Gly Gly Ile Gln Ser Ile Leu Gly Ser Val Leu Ser Gly Val
145             150             155             160

Asn Leu Asn Glu Leu Leu Gln Asn Lys Asp Pro Asn Gln Leu Glu Leu
            165             170             175

Ala Lys Ala Gly Leu Glu Leu Thr Asn Glu Leu Val Gly Asn Ile Ala
            180             185             190

Ser Ser Val Gln Thr Val Asp Ala Phe Ala Glu Gln Ile Ser Lys Leu
            195             200             205

Gly Ser His Leu Gln Asn Val Lys Gly Leu Gly Gly Leu Ser Asn Lys
    210             215             220

Leu Gln Asn Leu Pro Asp Leu Gly Lys Ala Ser Leu Gly Leu Asp Ile
225             230             235             240

Ile Ser Gly Leu Leu Ser Gly Ala Ser Ala Gly Leu Ile Leu Ala Asp
            245             250             255

Lys Glu Ala Ser Thr Glu Lys Lys Ala Ala Ala Gly Val Glu Phe Ala
            260             265             270

Asn Gln Ile Ile Gly Asn Val Thr Lys Ala Val Ser Ser Tyr Ile Leu
            275             280             285

Ala Gln Arg Val Ala Ser Gly Leu Ser Ser Thr Gly Pro Val Ala Ala
            290             295             300

Leu Ile Ala Ser Thr Val Ala Leu Ala Val Ser Pro Leu Ser Phe Leu
305             310             315             320

Asn Val Ala Asp Lys Phe Lys Gln Ala Asp Leu Ile Lys Ser Tyr Ser
            325             330             335

Glu Arg Phe Gln Lys Leu Gly Tyr Asp Gly Asp Arg Leu Leu Ala Asp
            340             345             350

Phe His Arg Glu Thr Gly Thr Ile Asp Ala Ser Val Thr Thr Ile Asn
            355             360             365

Thr Ala Leu Ala Ala Ile Ser Gly Gly Val Gly Ala Ala Ser Ala Gly
370             375             380

Ser Leu Val Gly Ala Pro Val Ala Leu Leu Val Ala Gly Val Thr Gly
385             390             395             400

Leu Ile Thr Thr Ile Leu Glu Tyr Ser Lys Gln Ala Met Phe Glu His
405             410             415

Val Ala Asn Lys Val His Asp Arg Ile Val Glu Trp Glu Lys Lys His
420             425             430

Asn Lys Asn Tyr Phe Glu Gln Gly Tyr Asp Ser Arg His Leu Ala Asp
435             440             445

Leu Gln Asp Asn Met Lys Phe Leu Ile Asn Leu Asn Lys Glu Leu Gln
450             455             460

Ala Glu Arg Val Val Ala Ile Thr Gln Gln Arg Trp Asp Asn Gln Ile
465             470             475             480

Gly Asp Leu Ala Ala Ile Ser Arg Arg Thr Asp Lys Ile Ser Ser Gly
485             490             495

Lys Ala Tyr Val Asp Ala Phe Glu Glu Gly Gln His Gln Ser Tyr Asp
500             505             510

Ser Ser Val Gln Leu Asp Asn Lys Asn Gly Ile Ile Asn Ile Ser Asn
515             520             525

Thr Asn Arg Lys Thr Gln Ser Val Leu Phe Arg Thr Pro Leu Leu Thr
530             535             540

Pro Gly Glu Glu Asn Arg Glu Arg Ile Gln Glu Gly Lys Asn Ser Tyr
545             550             555             560

Ile Thr Lys Leu His Ile Gln Arg Val Asp Ser Trp Thr Val Thr Asp
565             570             575

Gly Asp Ala Ser Ser Ser Val Asp Phe Thr Asn Val Val Gln Arg Ile
580             585             590

Ala Val Lys Phe Asp Asp Ala Gly Asn Ile Ile Glu Ser Lys Asp Thr
595             600             605

Lys Ile Ile Ala Asn Leu Gly Ala Gly Asn Asp Asn Val Phe Val Gly
610             615             620

27

Ser Ser Thr Thr Val Ile Asp Gly Gly Asp Gly His Asp Arg Val His
625                 630             635             640

Tyr Ser Arg Gly Glu Tyr Gly Ala Leu Val Ile Asp Ala Thr Ala Glu
                645             650             655

Thr Glu Lys Gly Ser Tyr Ser Val Lys Arg Tyr Val Gly Asp Ser Lys
            660             665             670

Ala Leu His Glu Thr Ile Ala Thr His Pro Thr Asn Val Gly Asn Arg
        675             680             685

Glu Glu Lys Ile Glu Tyr Arg Arg Glu Asp Asp Arg Phe His Thr Gly
        690             695             700

Tyr Thr Val Thr Asp Ser Leu Lys Ser Val Glu Glu Ile Ile Gly Ser
705             710             715             720

Gln Phe Asn Asp Ile Phe Lys Gly Ser Gln Phe Asp Asp Val Phe His
            725             730             735

Gly Gly Asn Gly Val Asp Thr Ile Asp Gly Asn Asp Gly Asp Asp His
            740             745             750

Leu Phe Gly Gly Ala Gly Asp Asp Val Ile Asp Gly Gly Asn Gly Asn
        755             760             765

Asn Phe Leu Val Gly Gly Thr Gly Asn Asp Ile Ile Ser Gly Gly Lys
    770             775             780

Asp Asn Asp Ile Tyr Val His Lys Thr Gly Asp Gly Asn Asp Ser Ile
785             790             795             800

Thr Asp Ser Gly Gly Gln Asp Lys Leu Ala Phe Ser Asp Val Asn Leu
            805             810             815

Lys Asp Leu Thr Phe Lys Lys Val Asp Ser Ser Leu Glu Ile Ile Asn
        820             825             830

Gln Lys Gly Glu Lys Val Arg Ile Gly Asn Trp Phe Leu Lys Asn Asp
        835             840             845

Leu Ala Ser Thr Val Ala Asn Tyr Lys Ala Thr Asn Asp Arg Lys Ile
    850             855             860

Glu Glu Ile Ile Gly Lys Gly Gly Glu Arg Ile Thr Ser Lys Gln Val
865             870             875             880

Asp Lys Leu Ile Lys Glu Gly Asn Asn Gln Ile Ser Ala Lys Ala Leu
                885                 890                 895

Ser Lys Val Gly Asn Asp Tyr Asn Thr Ser Lys Asp Arg Gln Asn Val
                900                 905                 910

Ser Asn Ser Leu Ala Lys Leu Ile Ser Ser Val Glu Ser Phe Thr Ser
                915                 920                 925

Ser Ser Asn Phe Arg Asn Asn Leu Gly Ala Tyr Val Pro Ser Ser Ile
            930                 935                 940

Asn Val Ser Asn Asn Ile Gln Leu Ala Arg Ala Ala
945                 950                 955


<210>  10
<211>  72
<212>  PRT
<213>  Actinobacillus pleuropneumoniae

<400>  10

Gly Asn Gly Val Asp Thr Ile Asp Gly Asn Asp Gly Asp Asp His Leu
1                   5                   10                  15

Phe Gly Gly Ala Gly Asp Asp Val Ile Asp Gly Gly Asn Gly Asn Asn
                20                  25                  30

Phe Leu Val Gly Gly Thr Gly Asn Asp Ile Ile Ser Gly Gly Lys Asp
            35                  40                  45

Asn Asp Ile Tyr Val His Lys Thr Gly Asp Gly Asn Asp Ser Ile Thr
            50                  55                  60

Asp Ser Gly Gly Gln Asp Lys Leu
65                  70


<210>  11
<211>  15
<212>  PRT
<213>  Actinobacillus pleuropneumoniae

<400>  11

Lys Val Gly Asn Asp Tyr Asn Thr Ser Lys Asp Arg Gln Asn Val
1                   5                   10                  15


<210>  12
<211>  41
<212>  PRT

<213>   Actinobacillus pleuropneumoniae

<400>   12

Leu Thr Pro Gly Glu Glu Asn Arg Glu Arg Ile Gln Glu Gly Lys Asn
1               5                   10                  15

Ser Tyr Ile Thr Lys Leu His Ile Gln Arg Val Asp Ser Trp Thr Val
            20                  25                  30

Thr Asp Gly Asp Ala Ser Ser Ser Val
        35                  40

<210>   13
<211>   20
<212>   PRT
<213>   Actinobacillus pleuropneumoniae

<400>   13

Ile Leu Tyr Ile Pro Gln Gly Tyr Asp Ser Gly Gln Gly Asn Gly Val
1               5                   10                  15

Gln Asp Leu Val
            20

<210>   14
<211>   24
<212>   PRT
<213>   Actinobacillus pleuropneumoniae

<400>   14

Ala Thr His Pro Thr Asn Val Gly Asn Arg Glu Glu Lys Ile Glu Tyr
1               5                   10                  15

Arg Arg Glu Asp Asp Arg Phe His
            20

<210>   15
<211>   410
<212>   PRT
<213>   Artificial Sequence

<220>
<223>   Recombinant ApxII toxin

<400>   15

Gly Asn Gly Val Asp Thr Ile Asp Gly Asn Asp Gly Asp Asp His Leu
1               5                   10                  15

Phe Gly Gly Ala Gly Asp Asp Val Ile Asp Gly Gly Asn Gly Asn Asn
            20                  25                  30

30

```
Phe Leu Val Gly Gly Thr Gly Asn Asp Ile Ile Ser Gly Gly Lys Asp
        35                40                45

Asn Asp Ile Tyr Val His Lys Thr Gly Asp Gly Asn Asp Ser Ile Thr
        50                55                60

Asp Ser Gly Gly Gln Asp Lys Leu Gly Ser Thr Ala Lys Val Gly Asn
65                70                75                80

Asp Tyr Asn Thr Ser Lys Asp Arg Gln Asn Val Gly Ser Thr Ala Gly
                85                90                95

Ser Thr Ala Leu Thr Pro Gly Glu Glu Asn Arg Glu Arg Ile Gln Glu
            100                105                110

Gly Lys Asn Ser Tyr Ile Thr Lys Leu His Ile Gln Arg Val Asp Ser
            115                120                125

Trp Thr Val Thr Asp Gly Asp Ala Ser Ser Ser Val Gly Ser Thr Ala
        130                135                140

Ile Leu Tyr Ile Pro Gln Gly Tyr Asp Ser Gly Gln Gly Asn Gly Val
145                150                155                160

Gln Asp Leu Val Gly Ser Thr Ala Ala Thr His Pro Thr Asn Val Gly
                165                170                175

Asn Arg Glu Glu Lys Ile Glu Tyr Arg Arg Glu Asp Asp Arg Phe His
                180                185                190

Gly Ser Thr Ala Gly Ser Thr Ala Phe Leu Asn Lys Leu Leu Ser Thr
        195                200                205

Ala Lys Glu Arg Asn Arg Trp Glu Glu Pro Gly Gln Lys Leu Tyr Asn
        210                215                220

Val Glu Ala Thr Ser Tyr Ala Gly Ser Thr Ala Phe Leu Asn Lys Leu
225                230                235                240

Leu Ser Thr Ala Lys Glu Arg Asn Arg Trp Glu Glu Pro Gly Gln Lys
            245                250                255

Leu Tyr Asn Val Glu Ala Thr Ser Tyr Ala Gly Ser Thr Ala Phe Leu
            260                265                270

Asn Lys Leu Leu Ser Thr Ala Lys Glu Arg Asn Arg Trp Glu Glu Pro
            275                280                285
```

```
Gly Gln Lys Leu Tyr Asn Val Glu Ala Thr Ser Tyr Ala Gly Ser Thr
    290             295             300

Ala Phe Leu Asn Lys Leu Leu Ser Thr Ala Lys Glu Arg Asn Arg Trp
305             310             315             320

Glu Glu Pro Gly Gln Lys Leu Tyr Asn Val Glu Ala Thr Ser Tyr Ala
            325             330             335

Gly Ser Thr Ala Phe Leu Asn Lys Leu Leu Ser Thr Ala Lys Glu Arg
        340             345             350

Asn Arg Trp Glu Glu Pro Gly Gln Lys Leu Tyr Asn Val Glu Ala Thr
        355             360             365

Ser Tyr Ala Gly Ser Thr Ala Phe Leu Asn Lys Leu Leu Ser Thr Ala
    370             375             380

Lys Glu Arg Asn Arg Trp Glu Glu Pro Gly Gln Lys Leu Tyr Asn Val
385             390             395             400

Glu Ala Thr Ser Tyr Ala Gly Ser Thr Ala
            405             410


<210>  16
<211>  412
<212>  PRT
<213>  Artificial Sequence

<220>
<223>  Recombinant ApxII toxin with a HindIII site

<400>  16

Gly Asn Gly Val Asp Thr Ile Asp Gly Asn Asp Gly Asp Asp His Leu
1               5               10              15

Phe Gly Gly Ala Gly Asp Asp Val Ile Asp Gly Gly Asn Gly Asn Asn
            20              25              30

Phe Leu Val Gly Gly Thr Gly Asn Asp Ile Ile Ser Gly Gly Lys Asp
        35              40              45

Asn Asp Ile Tyr Val His Lys Thr Gly Asp Gly Asn Asp Ser Ile Thr
    50              55              60

Asp Ser Gly Gly Gln Asp Lys Leu Gly Ser Thr Ala Lys Val Gly Asn
65              70              75              80
```

```
Asp Tyr Asn Thr Ser Lys Asp Arg Gln Asn Val Gly Ser Thr Ala Gly
                85              90              95

Ser Thr Ala Leu Thr Pro Gly Glu Glu Asn Arg Glu Arg Ile Gln Glu
            100             105             110

Gly Lys Asn Ser Tyr Ile Thr Lys Leu His Ile Gln Arg Val Asp Ser
            115             120             125

Trp Thr Val Thr Asp Gly Asp Ala Ser Ser Ser Val Gly Ser Thr Ala
    130             135             140

Ile Leu Tyr Ile Pro Gln Gly Tyr Asp Ser Gly Gln Gly Asn Gly Val
145             150             155             160

Gln Asp Leu Val Gly Ser Thr Ala Ala Thr His Pro Thr Asn Val Gly
            165             170             175

Asn Arg Glu Glu Lys Ile Glu Tyr Arg Arg Glu Asp Asp Arg Phe His
            180             185             190

Gly Ser Thr Ala Lys Leu Gly Ser Thr Ala Phe Leu Asn Lys Leu Leu
            195             200             205

Ser Thr Ala Lys Glu Arg Asn Arg Trp Glu Glu Pro Gly Gln Lys Leu
    210             215             220

Tyr Asn Val Glu Ala Thr Ser Tyr Ala Gly Ser Thr Ala Phe Leu Asn
225             230             235             240

Lys Leu Leu Ser Thr Ala Lys Glu Arg Asn Arg Trp Glu Glu Pro Gly
            245             250             255

Gln Lys Leu Tyr Asn Val Glu Ala Thr Ser Tyr Ala Gly Ser Thr Ala
            260             265             270

Phe Leu Asn Lys Leu Leu Ser Thr Ala Lys Glu Arg Asn Arg Trp Glu
            275             280             285

Glu Pro Gly Gln Lys Leu Tyr Asn Val Glu Ala Thr Ser Tyr Ala Gly
    290             295             300

Ser Thr Ala Phe Leu Asn Lys Leu Leu Ser Thr Ala Lys Glu Arg Asn
305             310             315             320

Arg Trp Glu Glu Pro Gly Gln Lys Leu Tyr Asn Val Glu Ala Thr Ser
            325             330             335
```

```
Tyr Ala Gly Ser Thr Ala Phe Leu Asn Lys Leu Leu Ser Thr Ala Lys
        340             345             350

Glu Arg Asn Arg Trp Glu Glu Pro Gly Gln Lys Leu Tyr Asn Val Glu
        355             360             365

Ala Thr Ser Tyr Ala Gly Ser Thr Ala Phe Leu Asn Lys Leu Leu Ser
370             375             380

Thr Ala Lys Glu Arg Asn Arg Trp Glu Glu Pro Gly Gln Lys Leu Tyr
385             390             395             400

Asn Val Glu Ala Thr Ser Tyr Ala Gly Ser Thr Ala
                405             410
```

```
<210>  17
<211>  1052
<212>  PRT
<213>  Actinobacillus pleuropneumoniae

<400>  17

Met Ser Thr Trp Ser Ser Met Leu Ala Asp Leu Lys Lys Arg Ala Glu
1               5               10              15

Glu Ala Lys Arg Gln Ala Lys Lys Gly Tyr Asp Val Thr Lys Asn Gly
        20              25              30

Leu Gln Tyr Gly Val Ser Gln Ala Lys Leu Gln Ala Leu Ala Ala Gly
        35              40              45

Lys Ala Val Gln Lys Tyr Gly Asn Lys Leu Val Leu Val Ile Pro Lys
    50              55              60

Glu Tyr Asp Gly Ser Val Gly Asn Gly Phe Phe Asp Leu Val Lys Ala
65              70              75              80

Ala Glu Glu Leu Gly Ile Gln Val Lys Tyr Val Asn Arg Asn Glu Leu
                85              90              95

Glu Val Ala His Lys Ser Leu Gly Thr Ala Asp Gln Phe Leu Gly Leu
                100             105             110

Thr Glu Arg Gly Leu Thr Leu Ser Ala Pro Gln Leu Asp Gln Phe Leu
        115             120             125

Gln Lys His Ser Lys Ile Ser Asn Val Val Gly Ser Ser Thr Gly Asp
        130             135             140
```

Ala Val Ser Lys Leu Ala Lys Ser Gln Thr Ile Ile Ser Gly Ile Gln
145             150             155             160

Ser Val Leu Gly Thr Val Leu Ala Gly Ile Asn Leu Asn Glu Ala Ile
            165             170             175

Ile Ser Gly Gly Ser Glu Leu Glu Leu Ala Glu Ala Gly Val Ser Leu
            180             185             190

Ala Ser Glu Leu Val Ser Asn Ile Ala Lys Gly Thr Thr Thr Ile Asp
            195             200             205

Ala Phe Thr Thr Gln Ile Gln Asn Phe Gly Lys Leu Val Glu Asn Ala
            210             215             220

Lys Gly Leu Gly Gly Val Gly Arg Gln Leu Gln Asn Ile Ser Gly Ser
225             230             235             240

Ala Leu Ser Lys Thr Gly Leu Gly Leu Asp Ile Ile Ser Ser Leu Leu
            245             250             255

Ser Gly Val Thr Ala Ser Phe Ala Leu Ala Asn Lys Asn Ala Ser Thr
            260             265             270

Ser Thr Lys Val Ala Ala Gly Phe Glu Leu Ser Asn Gln Val Ile Gly
            275             280             285

Gly Ile Thr Lys Ala Val Ser Ser Tyr Ile Leu Ala Gln Arg Leu Ala
            290             295             300

Ala Gly Leu Ser Thr Thr Gly Pro Ala Ala Ala Leu Ile Ala Ser Ser
305             310             315             320

Ile Ser Leu Ala Ile Ser Pro Leu Ala Phe Leu Arg Val Ala Asp Asn
            325             330             335

Phe Asn Arg Ser Lys Glu Ile Gly Glu Phe Ala Glu Arg Phe Lys Lys
            340             345             350

Leu Gly Tyr Asp Gly Asp Lys Leu Leu Ser Glu Phe Tyr His Glu Ala
            355             360             365

Gly Thr Ile Asp Ala Ser Ile Thr Thr Ile Ser Thr Ala Leu Ser Ala
            370             375             380

Ile Ala Ala Gly Thr Ala Ala Ala Ser Ala Gly Ala Leu Val Gly Ala

35

```
           385                          390                          395                          400


           Pro Ile Thr Leu Leu Val Thr Gly Ile Thr Gly Leu Ile Ser Gly Ile
                       405                  410                  415


           Leu Glu Phe Ser Lys Gln Pro Met Leu Asp His Val Ala Ser Lys Ile
                       420                  425                  430


           Gly Asn Lys Ile Asp Glu Trp Glu Lys Lys Tyr Gly Lys Asn Tyr Phe
                   435                  440                  445


           Glu Asn Gly Tyr Asp Ala Arg His Lys Ala Phe Leu Glu Asp Ser Phe
               450                  455                  460


           Ser Leu Leu Ser Ser Phe Asn Lys Gln Tyr Glu Thr Glu Arg Ala Val
           465                  470                  475                  480


           Leu Ile Thr Gln Gln Arg Trp Asp Glu Tyr Ile Gly Glu Leu Ala Gly
                       485                  490                  495


           Ile Thr Gly Lys Gly Asp Lys Leu Ser Ser Gly Lys Ala Tyr Val Asp
                   500                  505                  510


           Tyr Phe Gln Glu Gly Lys Leu Leu Glu Lys Lys Pro Asp Asp Phe Ser
                   515                  520                  525


           Lys Val Val Phe Asp Pro Thr Lys Gly Glu Ile Asp Ile Ser Asn Ser
               530                  535                  540


           Gln Thr Ser Thr Leu Leu Lys Phe Val Thr Pro Leu Leu Thr Pro Gly
           545                  550                  555                  560


           Thr Glu Ser Arg Glu Arg Thr Gln Thr Gly Lys Tyr Glu Tyr Ile Thr
                       565                  570                  575


           Lys Leu Val Val Lys Gly Lys Asp Lys Trp Val Val Asn Gly Val Lys
                   580                  585                  590


           Asp Lys Gly Ala Val Tyr Asp Tyr Thr Asn Leu Ile Gln His Ala His
                   595                  600                  605


           Ile Ser Ser Ser Val Ala Arg Gly Glu Glu Tyr Arg Glu Val Arg Leu
               610                  615                  620


           Val Ser His Leu Gly Asn Gly Asn Asp Lys Val Phe Leu Ala Ala Gly
           625                  630                  635                  640
```

```
Ser Ala Glu Ile His Ala Gly Glu Gly His Asp Val Val Tyr Tyr Asp
            645             650             655

Lys Thr Asp Thr Gly Leu Leu Val Ile Asp Gly Thr Lys Ala Thr Glu
            660             665             670

Gln Gly Arg Tyr Ser Val Thr Arg Glu Leu Ser Gly Ala Thr Lys Ile
            675             680             685

Leu Arg Glu Val Ile Lys Asn Gln Lys Ser Ala Val Gly Lys Arg Glu
            690             695             700

Glu Thr Leu Glu Tyr Arg Asp Tyr Glu Leu Thr Gln Ser Gly Asn Ser
705             710             715             720

Asn Leu Lys Ala His Asp Glu Leu His Ser Val Glu Glu Ile Ile Gly
            725             730             735

Ser Asn Gln Arg Asp Glu Phe Lys Gly Ser Lys Phe Arg Asp Ile Phe
            740             745             750

His Gly Ala Asp Gly Asp Asp Leu Leu Asn Gly Asn Asp Gly Asp Asp
            755             760             765

Ile Leu Tyr Gly Asp Lys Gly Asn Asp Glu Leu Arg Gly Asp Asn Gly
            770             775             780

Asn Asp Gln Leu Tyr Gly Gly Glu Gly Asn Asp Lys Leu Leu Gly Gly
785             790             795             800

Asn Gly Asn Asn Tyr Leu Ser Gly Gly Asp Gly Asn Asp Glu Leu Gln
            805             810             815

Val Leu Gly Asn Gly Phe Asn Val Leu Arg Ala Gly Lys Gly Asp Asp
            820             825             830

Lys Leu Tyr Gly Ser Ser Gly Ser Asp Leu Leu Asp Gly Gly Glu Gly
            835             840             845

Asn Asp Tyr Leu Glu Gly Gly Asp Gly Ser Asp Phe Tyr Val Tyr Arg
            850             855             860

Ser Thr Ser Gly Asn His Thr Ile Tyr Asp Gln Gly Lys Ser Ser Asp
865             870             875             880

Leu Asp Lys Leu Tyr Leu Ser Asp Phe Ser Phe Asp Arg Leu Leu Val
            885             890             895
```

37

```
Glu Lys Val Asp Asp Asn Leu Val Leu Arg Ser Asn Glu Ser Ser His
            900             905             910

Asn Asn Arg Val Leu Thr Ile Lys Asp Trp Phe Lys Glu Gly Asn Lys
            915             920             925

Tyr Asn His Lys Ile Glu Gln Ile Val Asp Lys Asn Gly Arg Lys Leu
            930             935             940

Thr Ala Glu Asn Leu Gly Thr Tyr Phe Lys Asn Ala Pro Lys Ala Asp
945             950             955             960

Asn Leu Leu Asn Tyr Ala Thr Lys Glu Asp Gln Asn Glu Ser Asn Leu
                965             970             975

Ser Ser Leu Lys Thr Glu Leu Ser Lys Ile Ile Thr Asn Ala Gly Asn
            980             985             990

Phe Gly Val Ala Lys Gln Gly Asn  Thr Gly Ile Asn Thr  Ala Ala Leu
            995             1000            1005

Asn Asn  Glu Val Asn Lys Ile  Ile Ser Ser Ala Asn  Thr Phe Ala
    1010            1015            1020

Thr Ser  Gln Leu Gly Gly Ser  Gly Met Gly Thr Leu  Pro Ser Thr
    1025            1030            1035

Asn Val  Asn Ser Met Met Leu  Gly Asn Leu Ala Arg  Ala Ala
    1040            1045            1050


<210>  18
<211>  127
<212>  PRT
<213>  Actinobacillus pleuropneumoniae

<400>  18

Ala Asp Gly Asp Asp Leu Leu Asn Gly Asn Asp Gly Asp Asp Ile Leu
1               5               10              15

Tyr Gly Asp Lys Gly Asn Asp Glu Leu Arg Gly Asp Asn Gly Asn Asp
            20              25              30

Gln Leu Tyr Gly Gly Glu Gly Asn Asp Lys Leu Leu Gly Gly Asn Gly
            35              40              45

Asn Asn Tyr Leu Ser Gly Gly Asp Gly Asn Asp Glu Leu Gln Val Leu
    50              55              60
```

```
Gly Asn Gly Phe Asn Val Leu Arg Ala Gly Lys Gly Asp Asp Lys Leu
65                  70                  75                  80


Tyr Gly Ser Ser Gly Ser Asp Leu Leu Asp Gly Gly Glu Gly Asn Asp
                85                  90                  95


Tyr Leu Glu Gly Gly Asp Gly Ser Asp Phe Tyr Val Tyr Arg Ser Thr
            100                 105                 110


Ser Gly Asn His Thr Ile Tyr Asp Gln Gly Lys Ser Ser Asp Leu
        115                 120                 125


<210>  19
<211>  80
<212>  PRT
<213>  Actinobacillus pleuropneumoniae

<400>  19

Gly Glu Leu Ala Gly Ile Thr Gly Lys Gly Asp Lys Leu Ser Ser Gly
1               5                   10                  15


Lys Ala Tyr Val Asp Tyr Phe Gln Glu Gly Lys Leu Leu Glu Lys Lys
            20                  25                  30


Pro Asp Asp Phe Ser Lys Val Val Phe Asp Pro Thr Lys Gly Glu Ile
            35                  40                  45


Asp Ile Ser Asn Ser Gln Thr Ser Thr Leu Leu Lys Phe Val Thr Pro
        50                  55                  60


Leu Leu Thr Pro Gly Thr Glu Ser Arg Glu Arg Thr Gln Thr Gly Lys
65                  70                  75                  80


<210>  20
<211>  433
<212>  PRT
<213>  Artificial Sequence

<220>
<223>  Recombinant ApxIII toxin

<400>  20

Ala Asp Gly Asp Asp Leu Leu Asn Gly Asn Asp Gly Asp Asp Ile Leu
1               5                   10                  15


Tyr Gly Asp Lys Gly Asn Asp Glu Leu Arg Gly Asp Asn Gly Asn Asp
            20                  25                  30


Gln Leu Tyr Gly Gly Glu Gly Asn Asp Lys Leu Leu Gly Gly Asn Gly
```

                35                          40                          45

Asn Asn Tyr Leu Ser Gly Gly Asp Gly Asn Asp Glu Leu Gln Val Leu
    50                  55                  60

Gly Asn Gly Phe Asn Val Leu Arg Ala Gly Lys Gly Asp Asp Lys Leu
65                  70                  75                      80

Tyr Gly Ser Ser Gly Ser Asp Leu Leu Asp Gly Gly Glu Gly Asn Asp
                85                  90                  95

Tyr Leu Glu Gly Gly Asp Gly Ser Asp Phe Tyr Val Tyr Arg Ser Thr
            100                 105                 110

Ser Gly Asn His Thr Ile Tyr Asp Gln Gly Lys Ser Ser Asp Leu Gly
            115                 120                 125

Ser Thr Ala Gly Ser Thr Ala Gly Glu Leu Ala Gly Ile Thr Gly Lys
    130                 135                 140

Gly Asp Lys Leu Ser Ser Gly Lys Ala Tyr Val Asp Tyr Phe Gln Glu
145                 150                 155                     160

Gly Lys Leu Leu Glu Lys Lys Pro Asp Asp Phe Ser Lys Val Val Phe
            165                 170                 175

Asp Pro Thr Lys Gly Glu Ile Asp Ile Ser Asn Ser Gln Thr Ser Thr
            180                 185                 190

Leu Leu Lys Phe Val Thr Pro Leu Leu Thr Pro Gly Thr Glu Ser Arg
            195                 200                 205

Glu Arg Thr Gln Thr Gly Lys Gly Ser Thr Ala Gly Ser Thr Ala Phe
    210                 215                 220

Leu Asn Lys Leu Leu Ser Thr Ala Lys Glu Arg Asn Arg Trp Glu Glu
225                 230                 235                     240

Pro Gly Gln Lys Leu Tyr Asn Val Glu Ala Thr Ser Tyr Ala Gly Ser
            245                 250                 255

Thr Ala Phe Leu Asn Lys Leu Leu Ser Thr Ala Lys Glu Arg Asn Arg
            260                 265                 270

Trp Glu Glu Pro Gly Gln Lys Leu Tyr Asn Val Glu Ala Thr Ser Tyr
    275                 280                 285

```
Ala Gly Ser Thr Ala Phe Leu Asn Lys Leu Leu Ser Thr Ala Lys Glu
    290             295             300

Arg Asn Arg Trp Glu Glu Pro Gly Gln Lys Leu Tyr Asn Val Glu Ala
305             310             315             320

Thr Ser Tyr Ala Gly Ser Thr Ala Phe Leu Asn Lys Leu Leu Ser Thr
            325             330             335

Ala Lys Glu Arg Asn Arg Trp Glu Glu Pro Gly Gln Lys Leu Tyr Asn
            340             345             350

Val Glu Ala Thr Ser Tyr Ala Gly Ser Thr Ala Phe Leu Asn Lys Leu
            355             360             365

Leu Ser Thr Ala Lys Glu Arg Asn Arg Trp Glu Glu Pro Gly Gln Lys
    370             375             380

Leu Tyr Asn Val Glu Ala Thr Ser Tyr Ala Gly Ser Thr Ala Phe Leu
385             390             395             400

Asn Lys Leu Leu Ser Thr Ala Lys Glu Arg Asn Arg Trp Glu Glu Pro
            405             410             415

Gly Gln Lys Leu Tyr Asn Val Glu Ala Thr Ser Tyr Ala Gly Ser Thr
            420             425             430

Ala
```

```
<210>  21
<211>  435
<212>  PRT
<213>  Artificial Sequence

<220>
<223>  Recombinant ApxIII with a HindIII site

<400>  21
```

```
Ala Asp Gly Asp Asp Leu Leu Asn Gly Asn Asp Gly Asp Asp Ile Leu
1               5               10              15

Tyr Gly Asp Lys Gly Asn Asp Glu Leu Arg Gly Asp Asn Gly Asn Asp
            20              25              30

Gln Leu Tyr Gly Gly Glu Gly Asn Asp Lys Leu Leu Gly Gly Asn Gly
            35              40              45

Asn Asn Tyr Leu Ser Gly Gly Asp Gly Asn Asp Glu Leu Gln Val Leu
```

                    50                        55                        60

Gly Asn Gly Phe Asn Val Leu Arg Ala Gly Lys Gly Asp Asp Lys Leu
65                  70              75                  80

Tyr Gly Ser Ser Gly Ser Asp Leu Leu Asp Gly Gly Glu Gly Asn Asp
                85              90                  95

Tyr Leu Glu Gly Gly Asp Gly Ser Asp Phe Tyr Val Tyr Arg Ser Thr
            100             105             110

Ser Gly Asn His Thr Ile Tyr Asp Gln Gly Lys Ser Ser Asp Leu Gly
        115             120             125

Ser Thr Ala Gly Ser Thr Ala Gly Glu Leu Ala Gly Ile Thr Gly Lys
        130             135             140

Gly Asp Lys Leu Ser Ser Gly Lys Ala Tyr Val Asp Tyr Phe Gln Glu
145             150             155             160

Gly Lys Leu Leu Glu Lys Lys Pro Asp Asp Phe Ser Lys Val Val Phe
            165             170             175

Asp Pro Thr Lys Gly Glu Ile Asp Ile Ser Asn Ser Gln Thr Ser Thr
        180             185             190

Leu Leu Lys Phe Val Thr Pro Leu Leu Thr Pro Gly Thr Glu Ser Arg
        195             200             205

Glu Arg Thr Gln Thr Gly Lys Gly Ser Thr Ala Lys Leu Gly Ser Thr
210             215             220

Ala Phe Leu Asn Lys Leu Leu Ser Thr Ala Lys Glu Arg Asn Arg Trp
225             230             235             240

Glu Glu Pro Gly Gln Lys Leu Tyr Asn Val Glu Ala Thr Ser Tyr Ala
            245             250             255

Gly Ser Thr Ala Phe Leu Asn Lys Leu Leu Ser Thr Ala Lys Glu Arg
        260             265             270

Asn Arg Trp Glu Glu Pro Gly Gln Lys Leu Tyr Asn Val Glu Ala Thr
        275             280             285

Ser Tyr Ala Gly Ser Thr Ala Phe Leu Asn Lys Leu Leu Ser Thr Ala
        290             295             300

Lys Glu Arg Asn Arg Trp Glu Glu Pro Gly Gln Lys Leu Tyr Asn Val
305                 310                 315                 320

Glu Ala Thr Ser Tyr Ala Gly Ser Thr Ala Phe Leu Asn Lys Leu Leu
                325                 330                 335

Ser Thr Ala Lys Glu Arg Asn Arg Trp Glu Glu Pro Gly Gln Lys Leu
            340                 345                 350

Tyr Asn Val Glu Ala Thr Ser Tyr Ala Gly Ser Thr Ala Phe Leu Asn
            355                 360                 365

Lys Leu Leu Ser Thr Ala Lys Glu Arg Asn Arg Trp Glu Glu Pro Gly
        370                 375                 380

Gln Lys Leu Tyr Asn Val Glu Ala Thr Ser Tyr Ala Gly Ser Thr Ala
385                 390                 395                 400

Phe Leu Asn Lys Leu Leu Ser Thr Ala Lys Glu Arg Asn Arg Trp Glu
            405                 410                 415

Glu Pro Gly Gln Lys Leu Tyr Asn Val Glu Ala Thr Ser Tyr Ala Gly
            420                 425                 430

Ser Thr Ala
            435


<210>   22
<211>   31
<212>   PRT
<213>   Sus scrofa

<300>
<308>   GenBank: ADG26759
<309>   2010-05-08
<313>   (201)..(231)

<400>   22

Phe Leu Asn Lys Leu Leu Ser Thr Ala Lys Glu Arg Asn Arg Trp Glu
1                   5                   10                  15

Glu Pro Gly Gln Lys Leu Tyr Asn Val Glu Ala Thr Ser Tyr Ala
            20                  25                  30

<210>   23
<211>   296
<212>   PRT
<213>   Sus scrofa

<300>
<308>   GenBank: ADG26759

<309>  2010-05-08
<313>  (1)..(296)

<400>  23

Thr Pro Ser Gly Cys Gly Lys Gln Asn Met Ile Gly Met Thr Pro Thr
1                   5                   10                  15

Val Ile Ala Val His Tyr Leu Asp Ser Thr Glu Gln Trp Glu Lys Phe
                20                  25                  30

Gly Leu Glu Lys Arg Gln Glu Ala Leu Glu Leu Ile Lys Lys Gly Tyr
            35                  40                  45

Thr Gln Gln Leu Ala Phe Arg Gln Lys Asn Ser Ala Phe Ala Ala Phe
        50                  55                  60

Gln Asp Arg Leu Ser Ser Thr Trp Leu Thr Ala Tyr Val Val Lys Val
65                  70                  75                  80

Phe Ala Met Ala Ala Asn Leu Ile Ala Ile Asp Ser Gln Val Leu Cys
                85                  90                  95

Gly Ala Val Lys Trp Leu Ile Leu Glu Lys Gln Lys Pro Asp Gly Val
            100                 105                 110

Phe Glu Glu Asn Gly Pro Val Ile His Gln Glu Met Ile Gly Gly Phe
        115                 120                 125

Lys Asn Thr Glu Glu Lys Asp Val Ser Leu Thr Ala Phe Val Leu Ile
    130                 135                 140

Ala Leu Gln Glu Ala Lys Asp Ile Cys Glu Pro Gln Val Asn Ser Leu
145                 150                 155                 160

Leu Arg Ser Ile Asn Lys Ala Arg Asp Phe Leu Ala Asp Tyr Tyr Leu
                165                 170                 175

Glu Leu Lys Arg Pro Tyr Thr Val Ala Ile Ala Gly Tyr Ala Leu Ala
            180                 185                 190

Leu Ser Asp Lys Leu Asp Glu Pro Phe Leu Asn Lys Leu Leu Ser Thr
        195                 200                 205

Ala Lys Glu Arg Asn Arg Trp Glu Glu Pro Gly Gln Lys Leu Tyr Asn
    210                 215                 220

Val Glu Ala Thr Ser Tyr Ala Leu Leu Ala Leu Leu Val Val Lys Asp
225                 230                 235                 240

```
Phe Asp Ser Val Pro Pro Ile Val Arg Trp Leu Asn Glu Gln Arg Tyr
                245             250             255

Tyr Gly Gly Gly Tyr Gly Ser Thr Gln Ala Thr Phe Met Val Phe Gln
            260             265             270

Ala Leu Ala Gln Tyr Gln Lys Asp Val Pro Asp His Lys Asp Leu Asn
        275             280             285

Leu Asp Val Ser Ile His Leu Pro
    290             295


<210>  24
<211>  28
<212>  PRT
<213>  Mus musculus

<300>
<308>  GenBank: ADL70201
<309>  2010-08-18
<313>  (211)..(238)

<400>  24

Lys Phe Leu Asn Thr Ala Lys Asp Arg Asn Arg Trp Glu Glu Pro Asp
1               5               10              15

Gln Gln Leu Tyr Asn Val Glu Ala Thr Ser Tyr Ala
            20              25


<210>  25
<211>  312
<212>  PRT
<213>  Mus musculus

<300>
<308>  GenBank: ADL70201
<309>  2010-08-18
<313>  (1)..(312)

<400>  25

Arg Leu Lys His Leu Ile Val Thr Pro Ala Gly Cys Gly Glu Gln Asn
1               5               10              15

Met Ile Gly Met Thr Pro Thr Val Ile Ala Val His Tyr Leu Asp Gln
            20              25              30

Thr Glu Gln Trp Glu Lys Phe Gly Ile Glu Lys Arg Gln Glu Ala Leu
        35              40              45

Glu Leu Ile Lys Lys Gly Tyr Thr Gln Gln Leu Ala Phe Lys Gln Pro
```

50                          55                          60

Ser Ser Ala Tyr Ala Ala Phe Asn Asn Arg Pro Pro Ser Thr Trp Leu
65              70              75                  80

Thr Ala Tyr Val Val Lys Val Phe Ser Leu Ala Ala Asn Leu Ile Ala
            85              90                  95

Ile Asp Ser His Val Leu Cys Gly Ala Val Lys Trp Leu Ile Leu Glu
            100             105             110

Lys Gln Lys Pro Asp Gly Val Phe Gln Glu Asp Gly Pro Val Ile His
        115             120             125

Gln Glu Met Ile Gly Gly Phe Arg Asn Ala Lys Glu Ala Asp Val Ser
        130             135             140

Leu Thr Ala Phe Val Leu Ile Ala Leu Gln Glu Ala Arg Asp Ile Cys
145             150             155             160

Glu Gly Gln Val Asn Ser Leu Pro Gly Ser Ile Asn Lys Ala Gly Glu
            165             170             175

Tyr Ile Glu Ala Ser Tyr Met Asn Leu Gln Arg Pro Tyr Thr Val Ala
        180             185             190

Ile Ala Gly Tyr Ala Leu Ala Leu Met Asn Lys Leu Glu Glu Pro Tyr
        195             200             205

Leu Gly Lys Phe Leu Asn Thr Ala Lys Asp Arg Asn Arg Trp Glu Glu
        210             215             220

Pro Asp Gln Gln Leu Tyr Asn Val Glu Ala Thr Ser Tyr Ala Leu Leu
225             230             235             240

Ala Leu Leu Leu Leu Lys Asp Phe Asp Ser Val Pro Pro Val Val Arg
            245             250             255

Trp Leu Asn Glu Gln Arg Tyr Tyr Gly Gly Gly Tyr Gly Ser Thr Gln
            260             265             270

Ala Thr Phe Met Val Phe Gln Ala Leu Ala Gln Tyr Gln Thr Asp Val
        275             280             285

Pro Asp His Lys Asp Leu Asn Met Asp Val Ser Phe His Leu Pro Ser
        290             295             300

```
Cys Ser Ser Ala Thr Thr Phe Arg
305                 310
```

```
<210>   26
<211>   4
<212>   PRT
<213>   Artificial Sequence

<220>
<223>   Amino acid sequence of GSTA linker

<400>   26
```

```
Gly Ser Thr Ala
1
```

```
<210>   27
<211>   9
<212>   PRT
<213>   Artificial Sequence

<220>
<223>   Amino acid sequence of GS linker

<400>   27
```

```
Gly Gly Gly Gly Ser Gly Gly Gly Gly
1                   5
```

```
<210>   28
<211>   14
<212>   PRT
<213>   Artificial Sequence

<220>
<223>   Amino acid sequence of GSGGG linker

<400>   28
```

```
Gly Ser Gly Gly Gly Gly Ser Gly Gly Gly Gly Ser Gly Ser
1                   5                   10
```

```
<210>   29
<211>   6
<212>   PRT
<213>   Artificial Sequence

<220>
<223>   Amino acid sequence of (GS)3 linker

<400>   29
```

```
Gly Ser Gly Ser Gly Ser
1                   5
```

```
<210>   30
<211>   15
<212>   PRT
```

<210> Artificial Sequence

<220>
<223> amino acid sequence of (GGGGS)3 linker

<400> 30

Gly Gly Gly Gly Ser Gly Gly Gly Gly Ser Gly Gly Gly Gly Ser
1               5                   10                  15


<210> 31
<211> 4
<212> PRT
<213> Artificial Sequence

<220>
<223> amino acid sequence of GGSG linker

<400> 31

Gly Gly Ser Gly
1


<210> 32
<211> 8
<212> PRT
<213> Artificial Sequence

<220>
<223> Amino acid sequence of (GGSG)2 linker

<400> 32

Gly Gly Ser Gly Gly Gly Ser Gly
1               5


<210> 33
<211> 12
<212> PRT
<213> Artificial Sequence

<220>
<223> amino acid sequence of (GGSG)3 linker

<400> 33

Gly Gly Ser Gly Gly Gly Ser Gly Gly Gly Ser Gly
1               5                   10


<210> 34
<211> 10
<212> PRT
<213> Artificial Sequence

<220>
<223> amino acid sequence of GENLYFQSGG linker

<400> 34

Gly Glu Asn Leu Tyr Phe Gln Ser Gly Gly
1               5                   10


<210> 35
<211> 36
<212> PRT
<213> Artificial Sequence

<220>
<223> amino acid sequence of GAST linker

<400> 35

Gly Gly Ser Ala Gly Gly Ser Gly Ser Gly Ser Ser Gly Gly Ser Ser
1               5                   10                  15


Gly Ala Ser Gly Thr Gly Thr Ala Gly Gly Thr Gly Ser Gly Ser Gly
            20              25              30


Thr Gly Ser Gly
            35


<210> 36
<211> 36
<212> PRT
<213> Artificial Sequence

<220>
<223> amino acid sequence of SEG linker

<400> 36

Gly Gly Ser Gly Gly Gly Ser Glu Gly Gly Gly Ser Glu Gly Gly Gly
1               5                   10                  15


Ser Glu Gly Gly Gly Ser Glu Gly Gly Gly Ser Glu Gly Gly Gly Ser
            20              25              30


Gly Gly Gly Ser
            35


<210> 37
<211> 10
<212> PRT
<213> Actinobacillus pleuropneumoniae

<400> 37

Arg Lys Gly Ala Asp Ala Lys Ser Gly Lys
1               5                   10


<210> 38
<211> 1951
<212> PRT
<213> Actinobacillus pleuropneumoniae

<400> 38

Met Thr Lys Leu Thr Met Gln Asp Val Thr Asn Leu Tyr Leu Tyr Lys
1               5                   10                  15

Thr Lys Thr Leu Pro Lys Asp Arg Leu Asp Asp Ser Leu Ile Ser Glu
            20                  25                  30

Ile Gly Lys Gly Asp Asp Asp Ile Asp Arg Lys Glu Phe Met Val Gly
            35                  40                  45

Pro Gly Arg Phe Val Thr Ala Asp Asn Phe Ser Val Val Arg Asp Phe
        50                  55                  60

Phe Asn Ala Gly Lys Ser Arg Ile Ile Ala Pro Gln Val Pro Pro Ile
65                  70                  75                  80

Arg Ser Gln Gln Glu Lys Ile Leu Val Gly Leu Lys Pro Gly Lys Tyr
            85                  90                  95

Ser Lys Ala Gln Ile Leu Glu Met Leu Gly Tyr Thr Lys Gly Gly Glu
            100                 105                 110

Val Val Asn Gly Met Phe Ala Gly Glu Val Gln Thr Leu Gly Phe Tyr
        115                 120                 125

Asp Asp Gly Lys Gly Asp Leu Leu Glu Arg Ala Tyr Ile Trp Asn Thr
        130                 135                 140

Thr Gly Phe Lys Met Ser Asp Asn Ala Phe Phe Val Ile Glu Glu Ser
145                 150                 155                 160

Gly Lys Arg Tyr Ile Glu Asn Phe Gly Ile Glu Pro Leu Gly Lys Gln
                165                 170                 175

Glu Asp Phe Asp Phe Val Gly Gly Phe Trp Ser Asn Leu Val Asn Arg
            180                 185                 190

Gly Leu Glu Ser Ile Ile Asp Pro Ser Gly Ile Gly Gly Thr Val Asn
        195                 200                 205

Leu Asn Phe Thr Gly Glu Val Glu Thr Tyr Thr Leu Asp Glu Thr Arg
        210                 215                 220

Phe Lys Ala Glu Ala Ala Lys Lys Ser His Trp Ser Leu Val Asn Ala
225                 230                 235                 240

```
Ala Lys Val Tyr Gly Gly Leu Asp Gln Ile Ile Lys Lys Leu Trp Asp
            245             250             255

Ser Gly Ser Ile Lys His Leu Tyr Gln Asp Lys Asp Thr Gly Lys Leu
            260             265             270

Lys Pro Ile Ile Tyr Gly Thr Ala Gly Asn Asp Ser Lys Ile Glu Gly
            275             280             285

Thr Lys Ile Thr Arg Arg Ile Ala Gly Lys Glu Val Thr Leu Asp Ile
            290             295             300

Ala Asn Gln Lys Ile Glu Lys Gly Val Leu Glu Lys Leu Gly Leu Ser
305             310             315             320

Val Ser Gly Ser Asp Ile Ile Lys Leu Leu Phe Gly Ala Leu Thr Pro
            325             330             335

Thr Leu Asn Arg Met Leu Leu Ser Gln Leu Ile Gln Ser Phe Ser Asp
            340             345             350

Ser Leu Ala Lys Leu Asp Asn Pro Leu Ala Pro Tyr Thr Lys Asn Gly
            355             360             365

Val Val Tyr Val Thr Gly Lys Gly Asn Asp Val Leu Lys Gly Thr Glu
            370             375             380

His Glu Asp Leu Phe Leu Gly Gly Glu Gly Asn Asp Thr Tyr Tyr Ala
385             390             395             400

Arg Val Gly Asp Thr Ile Glu Asp Ala Asp Gly Lys Gly Lys Val Tyr
            405             410             415

Phe Val Arg Glu Lys Gly Val Pro Lys Ala Asp Pro Lys Arg Val Glu
            420             425             430

Phe Ser Glu Tyr Ile Thr Lys Glu Glu Ile Lys Glu Val Glu Lys Gly
            435             440             445

Leu Leu Thr Tyr Ala Val Leu Glu Asn Tyr Asn Trp Glu Glu Lys Thr
            450             455             460

Ala Thr Phe Ala His Ala Thr Met Leu Asn Glu Leu Phe Thr Asp Tyr
465             470             475             480

Thr Asn Tyr Arg Tyr Glu Val Lys Gly Leu Lys Leu Pro Ala Val Lys
            485             490             495
```

```
Lys Leu Lys Ser Pro Leu Val Glu Phe Thr Ala Asp Leu Leu Thr Val
            500                 505                 510

Thr Pro Ile Asp Glu Asn Gly Lys Ala Leu Ser Glu Lys Ser Ile Thr
            515                 520                 525

Val Lys Asn Phe Lys Asn Gly Asp Leu Gly Ile Arg Leu Leu Asp Pro
            530                 535                 540

Asn Ser Tyr Tyr Tyr Phe Leu Glu Gly Lys Asp Thr Gly Phe Tyr Gly
545                 550                 555                 560

His Ala Phe Tyr Ile Glu Arg Lys Asn Gly Gly Gly Ser Lys Asn Asn
            565                 570                 575

Ser Ser Gly Ala Gly Asn Ser Lys Asp Trp Gly Gly Asn Gly His Gly
            580                 585                 590

Asn His Arg Asn Asn Ala Ser Asp Leu Asn Lys Pro Asp Gly Asn Asn
            595                 600                 605

Gly Asn Asn Gln Asn Asn Gly Ser Asn Gln Asp Asn Asn Ser Asp Val
            610                 615                 620

Asn Ala Pro Asn Asn Pro Gly Arg Asn Tyr Asp Ile Tyr Asp Pro Leu
625                 630                 635                 640

Ala Leu Asp Leu Asp Gly Asp Gly Leu Glu Thr Val Ser Met Asn Gly
            645                 650                 655

Arg Gln Gly Ala Leu Phe Asp His Glu Gly Lys Gly Ile Arg Thr Ala
            660                 665                 670

Thr Gly Trp Leu Ala Ala Asp Asp Gly Phe Leu Val Leu Asp Arg Asn
            675                 680                 685

Gln Asp Gly Ile Ile Asn Asp Ile Ser Glu Leu Phe Ser Asn Lys Asn
            690                 695                 700

Gln Leu Ser Asp Gly Ser Ile Ser Ala His Gly Phe Ala Ala Leu Ala
705                 710                 715                 720

Asp Leu Asp Thr Asn Gln Asp Gln Arg Ile Asp Gln Asn Asp Lys Leu
            725                 730                 735

Phe Ser Lys Leu Gln Ile Trp Arg Asp Leu Asn Gln Asn Gly Phe Ser
            740                 745                 750
```

52

Glu Ala Asn Glu Leu Phe Ser Leu Glu Ser Leu Asn Ile Lys Ser Leu
        755             760             765

His Thr Ala Tyr Glu Glu Arg Asn Asp Phe Leu Ala Gly Asn Asn Ile
        770             775             780

Leu Ala Gln Leu Gly Lys Tyr Glu Lys Thr Asp Gly Thr Phe Ala Gln
785             790             795             800

Met Gly Asp Leu Asn Phe Ser Phe Asn Pro Phe Tyr Ser Arg Phe Thr
            805             810             815

Glu Ala Leu Asn Leu Thr Glu Gln Gln Arg Arg Thr Ile Asn Leu Thr
        820             825             830

Gly Thr Gly Arg Val Arg Asp Leu Arg Glu Ala Ala Ala Leu Ser Glu
        835             840             845

Glu Leu Ala Ala Leu Leu Gln Gln Tyr Thr Lys Ala Ser Asp Phe Gln
        850             855             860

Ala Gln Arg Glu Leu Leu Pro Ala Ile Leu Asp Lys Trp Ala Ala Thr
865             870             875             880

Asp Leu Gln Tyr Gln His Tyr Asp Lys Thr Leu Leu Lys Thr Leu Glu
            885             890             895

Ser Thr Asp Ser Ser Ala Ser Val Val Arg Val Thr Pro Ser Gln Leu
            900             905             910

Ser Ser Ile Arg Asn Val Lys His Asp Pro Thr Val Met Gln Asn Phe
        915             920             925

Glu Gln Ser Lys Ala Lys Ile Ala Thr Leu Asn Ser Leu Tyr Gly Leu
        930             935             940

Asn Ile Asp Gln Leu Tyr Tyr Thr Thr Asp Lys Asp Ile Arg Tyr Ile
945             950             955             960

Thr Asp Lys Val Asn Asn Met Tyr Gln Thr Thr Val Glu Leu Ala Tyr
            965             970             975

Arg Ser Leu Leu Leu Gln Thr Arg Leu Lys Lys Tyr Val Tyr Ser Val
            980             985             990

Asn Ala Lys Gln Phe Glu Gly Lys  Trp Val Ala Asp Tyr  Ser Arg Thr

```
                    995                     1000                    1005

        Glu Ala  Leu Phe Asn Ser Thr  Phe Lys Gln Ser Pro  Glu Asn Ala
            1010            1015            1020

        Leu Tyr  Asp Leu Ser Glu Tyr  Leu Ser Phe Phe Asn  Asp Pro Thr
            1025            1030            1035

        Glu Trp  Lys Glu Gly Leu Leu  Leu Leu Ser Arg Tyr  Ile Asp Tyr
            1040            1045            1050

        Ala Lys  Ala Gln Gly Phe Tyr  Glu Asn Trp Ala Thr  Thr Ser Asn
            1055            1060            1065

        Leu Thr  Ile Ala Arg Leu Arg  Glu Ala Gly Val Ile  Phe Ala Glu
            1070            1075            1080

        Ser Thr  Asp Leu Lys Gly Asp  Glu Lys Asn Asn Ile  Leu Leu Gly
            1085            1090            1095

        Ser Gln  Lys Asp Asn Asn Leu  Ser Gly Ser Ala Gly  Asp Asp Leu
            1100            1105            1110

        Leu Ile  Gly Gly Glu Gly Asn  Asp Thr Leu Lys Gly  Ser Tyr Gly
            1115            1120            1125

        Ala Asp  Thr Tyr Leu Phe Ser  Lys Gly His Gly Gln  Asp Val Ile
            1130            1135            1140

        Tyr Glu  Tyr Ser Asp Ser Ala  Asn Ser Lys Ser Asp  Ile Asp Thr
            1145            1150            1155

        Leu Lys  Phe Thr Asp Ile Asn  Tyr Ala Glu Val Lys  Phe Arg Arg
            1160            1165            1170

        Val Gly  Asp Asp Leu Met Leu  Phe Gly Tyr His Asp  Thr Asp Ser
            1175            1180            1185

        Val Thr  Val Lys Ser Phe Tyr  Asn His Glu Tyr Tyr  Gln Phe Glu
            1190            1195            1200

        Lys Leu  Glu Phe Ala Asp Arg  Ser Ile Thr Arg Asp  Glu Leu Gly
            1205            1210            1215

        Lys Gln  Gly Met Ala Leu Phe  Gly Thr Asp Gly Asp  Asp Asp Ile
            1220            1225            1230
```

```
Asn Asp Trp Gly Arg Asn Ser  Val Ile Asp Ala Gly  Ala Gly Asn
    1235                1240                1245

Asp Thr Ile Asn Gly Ser Tyr  Gly Asp Asp Thr Leu  Ile Gly Gly
    1250                1255                1260

Thr Gly Asn Asp Ile Leu Lys  Gly Ser Tyr Gly Ala  Asp Thr Tyr
    1265                1270                1275

Leu Phe Ser Lys Gly His Gly  Gln Asp Val Ile Tyr  Glu Tyr Ser
    1280                1285                1290

Asp Ser Ala Asn Ser Lys Arg  Asp Ile Asp Thr Leu  Lys Phe Thr
    1295                1300                1305

Asp Val Asn Tyr Ala Glu Val  Lys Phe Arg Arg Val  Gly Asp Asp
    1310                1315                1320

Leu Met Leu Phe Gly Tyr His  Asp Thr Asp Ser Val  Thr Val Lys
    1325                1330                1335

Ser Phe Tyr Asp His Glu Tyr  Tyr Gln Phe Glu Lys  Leu Glu Phe
    1340                1345                1350

Ala Asp Arg Ser Ile Ser Arg  Asp Glu Leu Ile Lys  Ala Gly Leu
    1355                1360                1365

His Leu Tyr Gly Thr Asp Gly  Asn Asp Glu Ile Asn  Asp His Ala
    1370                1375                1380

Asp Trp Asp Ser Ile Leu Glu  Gly Gly Lys Gly Asn  Asp Ile Leu
    1385                1390                1395

Arg Gly Ser Tyr Gly Ala Asp  Thr Tyr Ile Phe Ser  Lys Gly His
    1400                1405                1410

Gly Gln Asp Val Ile Tyr Glu  Tyr Ser Asp Ser Ala  Asn Ser Lys
    1415                1420                1425

Arg Asp Ile Asp Thr Leu Lys  Phe Thr Asp Val Asn  Tyr Ala Glu
    1430                1435                1440

Val Lys Phe Arg Arg Val Asp  Asp Asp Leu Met Leu  Phe Gly Tyr
    1445                1450                1455

His Asp Thr Asp Ser Val Thr  Val Lys Ser Phe Tyr  Asp His Glu
    1460                1465                1470
```

```
Tyr Tyr Gln Phe Glu Lys Leu  Glu Phe Ala Asp Arg  Ser Ile Thr
    1475              1480              1485

Arg Asp Glu Leu Gly Lys Gln  Gly Met Ala Leu Phe  Gly Thr Asp
    1490              1495              1500

Gly Asp Asp Asp Ile Asn Asp  Trp Gly Arg Asn Ser  Val Ile Asp
    1505              1510              1515

Ala Gly Ala Gly Asn Asp Thr  Ile Asn Gly Gly Tyr  Gly Asp Asp
    1520              1525              1530

Thr Leu Ile Gly Gly Lys Gly  Asn Asp Ile Leu Lys  Gly Ser Tyr
    1535              1540              1545

Gly Ala Asp Thr Tyr Ile Phe  Ser Lys Gly His Gly  Gln Asp Ile
    1550              1555              1560

Val Tyr Glu Asp Thr Asn Asn  Asp Asn Arg Ala Arg  Asp Ile Asp
    1565              1570              1575

Thr Leu Lys Phe Thr Asp Val  Asn Tyr Ala Glu Val  Lys Phe Arg
    1580              1585              1590

Arg Val Asp Asn Asp Leu Met  Leu Phe Gly Tyr His  Asp Thr Asp
    1595              1600              1605

Ser Val Thr Val Lys Ser Phe  Tyr Ser His Val Asp  Tyr Gln Phe
    1610              1615              1620

Asp Lys Leu Glu Phe Ala Asp  Arg Ser Ile Thr Arg  Asp Glu Leu
    1625              1630              1635

Ile Lys Ala Gly Leu His Leu  Tyr Gly Thr Asp Gly  Asn Asp Asp
    1640              1645              1650

Ile Lys Asp His Ala Asp Trp  Asp Ser Ile Leu Glu  Gly Gly Lys
    1655              1660              1665

Gly Asn Asp Ile Leu Arg Gly  Gly Tyr Gly Ala Asp  Thr Tyr Ile
    1670              1675              1680

Phe Ser Lys Gly His Gly Gln  Asp Ile Val Tyr Glu  Asp Thr Asn
    1685              1690              1695

Asn Asp Asn Arg Ala Arg Asp  Ile Asp Thr Leu Lys  Phe Thr Asp
    1700              1705              1710
```

```
Val Asn Tyr Ala Glu Val Lys Phe Arg Arg Val Asp Asn Asp Leu
    1715            1720            1725

Met Leu Phe Gly Tyr His Asp Thr Asp Ser Val Thr Ile Lys Ser
    1730            1735            1740

Phe Tyr Asn His Val Asp Tyr Gln Phe Asp Lys Leu Asp Phe Ala
    1745            1750            1755

Asp Arg Ser Ile Thr Arg Asp Glu Leu Gly Lys Gln Gly Met Ala
    1760            1765            1770

Leu Phe Gly Thr Asp Gly Asp Asp Asn Ile Asn Asp Trp Gly Arg
    1775            1780            1785

Asn Ser Val Ile Asp Ala Gly Ala Gly Asn Asp Thr Val Asn Gly
    1790            1795            1800

Gly Asn Gly Asp Asp Thr Leu Ile Gly Gly Lys Gly Asn Asp Ile
    1805            1810            1815

Leu Arg Gly Gly Tyr Gly Ala Asp Thr Tyr Ile Phe Ser Lys Gly
    1820            1825            1830

His Gly Gln Asp Ile Val Tyr Glu Asp Thr Asn Asn Asp Asn Arg
    1835            1840            1845

Ala Arg Asp Ile Asp Thr Leu Lys Phe Thr Asp Ile Asn Leu Ser
    1850            1855            1860

Glu Leu Trp Phe Ser Arg Glu Asn Asn Asp Leu Ile Ile Lys Ser
    1865            1870            1875

Leu Leu Ser Glu Asp Lys Val Thr Val Gln Asn Trp Tyr Ser His
    1880            1885            1890

Gln Asp His Lys Ile Glu Asn Ile Arg Leu Ser Asn Glu Gln Thr
    1895            1900            1905

Leu Val Ser Thr Gln Val Glu Lys Met Val Glu Ser Met Ala Gly
    1910            1915            1920

Phe Ala Gln Lys His Gly Gly Glu Ile Ser Leu Ala Ser Pro Glu
    1925            1930            1935

Glu Val Lys Gln Tyr Ile Asn Ser Leu Thr Ala Ala Leu
```

```
<210>  39
<211>  16
<212>  PRT
<213>  Actinobacillus pleuropneumoniae

<400>  39

Gly Gly Pro Leu Ala Tyr Ser Asn Ser Pro Asn Ser Ile Pro Asn Ala
1               5                   10                  15


<210>  40
<211>  14
<212>  PRT
<213>  Actinobacillus pleuropneumoniae

<400>  40

Gly Ala Gly Gly Pro Leu Ala Tyr Ser Asn Ser Pro Asn Ser
1               5                   10


<210>  41
<211>  20
<212>  PRT
<213>  Actinobacillus pleuropneumoniae

<400>  41

Leu Val Gly Ala Gly Gly Pro Leu Ala Tyr Ser Asn Ser Pro Asn Ser
1               5                   10                  15

Ile Pro Asn Ala
            20


<210>  42
<211>  6
<212>  PRT
<213>  Actinobacillus pleuropneumoniae

<400>  42

Gly Ser Asn Arg Lys Asp
1               5


<210>  43
<211>  47
<212>  PRT
<213>  Actinobacillus pleuropneumoniae

<400>  43

Gly Ala Lys Gly Asp Asp Glu Ile Tyr Gly Asn Asp Gly His Asp Ile
1               5                   10                  15

Leu Tyr Gly Asp Asp Gly Asn Asp Val Ile His Gly Gly Asp Gly Asn
```

58

                    20                    25                    30

Asp His Leu Val Gly Gly Asn Gly Asn Asp Arg Leu Ile Gly Gly
        35                    40                    45


<210> 44
<211> 13
<212> PRT
<213> Actinobacillus pleuropneumoniae

<400> 44

Asn Asn Phe Leu Asn Gly Gly Asp Gly Asp Asp Glu Leu
1               5               10


<210> 45
<211> 20
<212> PRT
<213> Actinobacillus pleuropneumoniae

<400> 45

Gly Ser Arg Phe Thr Asp Ile Phe His Gly Ala Lys Gly Asp Asp Glu
1               5                   10                  15

Ile Tyr Gly Asn
            20


<210> 46
<211> 20
<212> PRT
<213> Actinobacillus pleuropneumoniae

<400> 46

Asp Val Ile His Gly Gly Asp Gly Asn Asp His Leu Val Gly Gly Asn
1               5                   10                  15

Gly Asn Asp Arg
            20


<210> 47
<211> 20
<212> PRT
<213> Actinobacillus pleuropneumoniae

<400> 47

Ile Gly Gly Lys Gly Asn Asn Phe Leu Asn Gly Gly Asp Gly Asp Asp
1               5                   10                  15

Glu Leu Gln Val
            20

<210> 48
<211> 14
<212> PRT
<213> Actinobacillus pleuropneumoniae

<400> 48

His Gly Ala Lys Gly Asp Asp Glu Ile Tyr Gly Asn Asp Gly
1               5                   10


<210> 49
<211> 14
<212> PRT
<213> Actinobacillus pleuropneumoniae

<400> 49

Gly Gly Lys Gly Asn Asn Phe Leu Asn Gly Gly Asp Gly Asp
1               5                   10


<210> 50
<211> 20
<212> PRT
<213> Actinobacillus pleuropneumoniae

<400> 50

Asp Gly His Asp Ile Leu Tyr Gly Asp Asp Gly Asn Asp Val Ile His
1               5                   10                  15

Gly Gly Asp Gly
            20


<210> 51
<211> 20
<212> PRT
<213> Actinobacillus pleuropneumoniae

<400> 51

Arg Leu Ile Gly Gly Lys Gly Asn Asn Phe Leu Asn Gly Gly Asp Gly
1               5                   10                  15

Asp Asp Glu Leu
            20


<210> 52
<211> 15
<212> PRT
<213> Actinobacillus pleuropneumoniae

<400> 52

Gly Thr Gly Asn Asp Ile Ile Ser Gly Gly Lys Asp Asn Asp Ile
1               5                   10                  15

<210> 53
<211> 19
<212> PRT
<213> Actinobacillus pleuropneumoniae

<400> 53

His Lys Thr Gly Asp Gly Asn Asp Ser Ile Thr Asp Ser Gly Gly Gln
1               5                   10                  15

Asp Lys Leu


<210> 54
<211> 20
<212> PRT
<213> Actinobacillus pleuropneumoniae

<400> 54

Phe Gly Gly Ala Gly Asp Asp Val Ile Asp Gly Gly Asn Gly Asn Asn
1               5                   10                  15

Phe Leu Val Gly
            20


<210> 55
<211> 20
<212> PRT
<213> Actinobacillus pleuropneumoniae

<400> 55

Tyr Val His Lys Thr Gly Asp Gly Asn Asp Ser Ile Thr Asp Ser Gly
1               5                   10                  15

Gly Gln Asp Lys
            20


<210> 56
<211> 14
<212> PRT
<213> Actinobacillus pleuropneumoniae

<400> 56

Gly Gly Ala Gly Asp Asp Val Ile Asp Gly Gly Asn Gly Asn
1               5                   10


<210> 57
<211> 14
<212> PRT
<213> Actinobacillus pleuropneumoniae

<400> 57

Gly Gly Thr Gly Asn Asp Ile Ile Ser Gly Gly Lys Asp Asn
1               5                   10

<210>  58
<211>  14
<212>  PRT
<213>  Actinobacillus pleuropneumoniae

<400>  58

Lys Thr Gly Asp Gly Asn Asp Ser Ile Thr Asp Ser Gly Gly
1               5                   10

<210>  59
<211>  20
<212>  PRT
<213>  Actinobacillus pleuropneumoniae

<400>  59

Ala Gly Asp Asp Val Ile Asp Gly Gly Asn Gly Asn Asn Phe Leu Val
1               5                   10                  15

Gly Gly Thr Gly
            20

<210>  60
<211>  20
<212>  PRT
<213>  Actinobacillus pleuropneumoniae

<400>  60

Asp Ile Tyr Val His Lys Thr Gly Asp Gly Asn Asp Ser Ile Thr Asp
1               5                   10                  15

Ser Gly Gly Gln
            20

<210>  61
<211>  17
<212>  PRT
<213>  Actinobacillus pleuropneumoniae

<400>  61

Leu Thr Pro Gly Glu Glu Asn Arg Glu Arg Ile Gln Glu Gly Lys Asn
1               5                   10                  15

Ser

<210>  62
<211>  12
<212>  PRT

<213> Actinobacillus pleuropneumoniae

<400> 62

Trp Thr Val Thr Asp Gly Asp Ala Ser Ser Ser Val
1               5                   10


<210> 63
<211> 20
<212> PRT
<213> Actinobacillus pleuropneumoniae

<400> 63

Thr Pro Gly Glu Glu Asn Arg Glu Arg Ile Gln Glu Gly Lys Asn Ser
1               5                   10                  15


Tyr Ile Thr Lys
                20


<210> 64
<211> 14
<212> PRT
<213> Actinobacillus pleuropneumoniae

<400> 64

Leu Phe Arg Thr Pro Leu Leu Thr Pro Gly Glu Glu Asn Arg
1               5                   10


<210> 65
<211> 12
<212> PRT
<213> Actinobacillus pleuropneumoniae

<400> 65

Gln Gly Tyr Asp Ser Gly Gln Gly Asn Gly Val Gln
1               5                   10


<210> 66
<211> 60
<212> PRT
<213> Actinobacillus pleuropneumoniae

<400> 66

Val Ile Asp Ala Gly Ala Gly Asn Asp Thr Val Asn Gly Gly Asn Gly
1               5                   10                  15


Asp Asp Thr Leu Ile Gly Gly Lys Gly Asn Asp Ile Leu Arg Gly Gly
            20                  25                  30


Tyr Gly Ala Asp Thr Tyr Ile Phe Ser Lys Gly His Gly Gln Asp Ile
            35                  40                  45

Val Tyr Glu Asp Thr Asn Asn Asp Asn Arg Ala Arg
    50                  55                  60


<210>  67
<211>  14
<212>  PRT
<213>  Actinobacillus pleuropneumoniae

<400>  67

Asn Arg Glu Glu Lys Ile Glu Tyr Arg Arg Glu Asp Asp Arg
1                   5                   10


<210>  68
<211>  20
<212>  PRT
<213>  Actinobacillus pleuropneumoniae

<400>  68

Pro Thr Asn Val Gly Asn Arg Glu Glu Lys Ile Glu Tyr Arg Arg Glu
1                   5                   10                  15


Asp Asp Arg Phe
            20


<210>  69
<211>  62
<212>  PRT
<213>  Actinobacillus pleuropneumoniae

<400>  69

Gly Ala Asp Gly Asp Asp Leu Leu Asn Gly Asn Asp Gly Asp Asp Ile
1                   5                   10                  15


Leu Tyr Gly Asp Lys Gly Asn Asp Glu Leu Arg Gly Asp Asn Gly Asn
            20                  25                  30


Asp Gln Leu Tyr Gly Gly Glu Gly Asn Asp Lys Leu Leu Gly Gly Asn
        35                  40                  45


Gly Asn Asn Tyr Leu Ser Gly Gly Asp Gly Asn Asp Glu Leu
    50                  55                  60


<210>  70
<211>  33
<212>  PRT
<213>  Actinobacillus pleuropneumoniae

<400>  70

Ala Gly Lys Gly Asp Asp Lys Leu Tyr Gly Ser Ser Gly Ser Asp Leu
1                   5                   10                  15

```
Leu Asp Gly Gly Glu Gly Asn Asp Tyr Leu Glu Gly Gly Asp Gly Ser
         20                  25                  30

Asp
```

```
<210>  71
<211>  16
<212>  PRT
<213>  Actinobacillus pleuropneumoniae

<400>  71

Ser Thr Ser Gly Asn His Thr Ile Tyr Asp Gln Gly Lys Ser Ser Asp
1               5                   10                  15
```

```
<210>  72
<211>  20
<212>  PRT
<213>  Actinobacillus pleuropneumoniae

<400>  72

Gly Asp Lys Gly Asn Asp Glu Leu Arg Gly Asp Asn Gly Asn Asp Gln
1               5                   10                  15

Leu Tyr Gly Gly
            20
```

```
<210>  73
<211>  20
<212>  PRT
<213>  Actinobacillus pleuropneumoniae

<400>  73

Leu Leu Gly Gly Asn Gly Asn Asn Tyr Leu Ser Gly Gly Asp Gly Asn
1               5                   10                  15

Asp Glu Leu Gln
            20
```

```
<210>  74
<211>  14
<212>  PRT
<213>  Actinobacillus pleuropneumoniae

<400>  74

Gly Asp Lys Gly Asn Asp Glu Leu Arg Gly Asp Asn Gly Asn
1               5                   10
```

```
<210>  75
```

```
<211>  14
<212>  PRT
<213>  Actinobacillus pleuropneumoniae

<400>  75

Gly Gly Asn Gly Asn Asn Tyr Leu Ser Gly Gly Asp Gly Asn
1               5                   10


<210>  76
<211>  20
<212>  PRT
<213>  Actinobacillus pleuropneumoniae

<400>  76

Asp Gly Asp Asp Ile Leu Tyr Gly Asp Lys Gly Asn Asp Glu Leu Arg
1               5                   10                  15

Gly Asp Asn Gly
            20


<210>  77
<211>  20
<212>  PRT
<213>  Actinobacillus pleuropneumoniae

<400>  77

Leu Leu Gly Gly Asn Gly Asn Asn Tyr Leu Ser Gly Gly Asp Gly Asn
1               5                   10                  15

Asp Glu Leu Gln
            20


<210>  78
<211>  20
<212>  PRT
<213>  Actinobacillus pleuropneumoniae

<400>  78

Asn Gly Phe Asn Val Leu Arg Ala Gly Lys Gly Asp Asp Lys Leu Tyr
1               5                   10                  15

Gly Ser Ser Gly
            20


<210>  79
<211>  14
<212>  PRT
<213>  Actinobacillus pleuropneumoniae

<400>  79

Gly Ile Thr Gly Lys Gly Asp Lys Leu Ser Ser Gly Lys Ala
```

```
                 1                5                    10
```

```
<210>   80
<211>   9
<212>   PRT
<213>   Actinobacillus pleuropneumoniae

<400>   80

Leu Leu Glu Lys Lys Pro Asp Asp Phe
1               5


<210>   81
<211>   15
<212>   PRT
<213>   Actinobacillus pleuropneumoniae

<400>   81

Phe Asp Pro Thr Lys Gly Glu Ile Asp Ile Ser Asn Ser Gln Thr
1               5                   10                  15


<210>   82
<211>   20
<212>   PRT
<213>   Actinobacillus pleuropneumoniae

<400>   82

Gly Ile Thr Gly Lys Gly Asp Lys Leu Ser Ser Gly Lys Ala Tyr Val
1               5                   10                  15

Asp Tyr Phe Gln
            20


<210>   83
<211>   20
<212>   PRT
<213>   Actinobacillus pleuropneumoniae

<400>   83

Val Phe Asp Pro Thr Lys Gly Glu Ile Asp Ile Ser Asn Ser Gln Thr
1               5                   10                  15

Ser Thr Leu Leu
            20


<210>   84
<211>   14
<212>   PRT
<213>   Actinobacillus pleuropneumoniae

<400>   84

Lys Gly Asp Lys Leu Ser Ser Gly Lys Ala Tyr Val Asp Tyr
```

EP 3 450 461 A1

1                  5                        10

<210> 85
<211> 14
<212> PRT
<213> Actinobacillus pleuropneumoniae

<400> 85

Glu Lys Lys Pro Asp Asp Phe Ser Lys Val Val Phe Asp Pro
1                  5                        10

<210> 86
<211> 14
<212> PRT
<213> Actinobacillus pleuropneumoniae

<400> 86

Phe Val Thr Pro Leu Leu Thr Pro Gly Thr Glu Ser Arg Glu
1                  5                        10

<210> 87
<211> 20
<212> PRT
<213> Actinobacillus pleuropneumoniae

<400> 87

Glu Leu Ala Gly Ile Thr Gly Lys Gly Asp Lys Leu Ser Ser Gly Lys
1                  5                        10                     15

Ala Tyr Val Asp
                20

<210> 88
<211> 20
<212> PRT
<213> Actinobacillus pleuropneumoniae

<400> 88

Thr Leu Leu Lys Phe Val Thr Pro Leu Leu Thr Pro Gly Thr Glu Ser
1                  5                        10                     15

Arg Glu Arg Thr
                20

<210> 89
<211> 84
<212> PRT
<213> Actinobacillus pleuropneumoniae

<400> 89

Glu Gly Lys Asp Thr Gly Phe Tyr Gly His Ala Phe Tyr Ile Glu Arg

```
        1              5                      10                     15


        Lys Asn Gly Gly Gly Ser Lys Asn Asn Ser Ser Gly Ala Gly Asn Ser
                    20                  25                  30


        Lys Asp Trp Gly Gly Asn Gly His Gly Asn His Arg Asn Asn Ala Ser
                    35                  40                  45


        Asp Leu Asn Lys Pro Asp Gly Asn Asn Gly Asn Asn Gln Asn Asn Gly
                50                  55                  60


        Ser Asn Gln Asp Asn Asn Ser Asp Val Asn Ala Pro Asn Asn Pro Gly
        65                  70                  75                  80


        Arg Asn Tyr Asp


        <210>   90
        <211>   66
        <212>   PRT
        <213>   Actinobacillus pleuropneumoniae

        <400>   90

        Val Ile Asp Ala Gly Ala Gly Asn Asp Thr Ile Asn Gly Gly Tyr Gly
        1               5                      10                  15


        Asp Asp Thr Leu Ile Gly Gly Lys Gly Asn Asp Ile Leu Lys Gly Ser
                    20                  25                  30


        Tyr Gly Ala Asp Thr Tyr Ile Phe Ser Lys Gly His Gly Gln Asp Ile
                    35                  40                  45


        Val Tyr Glu Asp Thr Asn Asn Asp Asn Arg Ala Arg Asp Ile Asp Thr
                50                  55                  60


        Leu Lys
        65


        <210>   91
        <211>   440
        <212>   PRT
        <213>   Artificial Sequence

        <220>
        <223>   Recombinant ApxIV toxin

        <400>   91

        Val Ile Asp Ala Gly Ala Gly Asn Asp Thr Val Asn Gly Gly Asn Gly
        1               5                      10                  15
```

```
Asp Asp Thr Leu Ile Gly Gly Lys Gly Asn Asp Ile Leu Arg Gly Gly
            20              25              30

Tyr Gly Ala Asp Thr Tyr Ile Phe Ser Lys Gly His Gly Gln Asp Ile
            35              40              45

Val Tyr Glu Asp Thr Asn Asn Asp Asn Arg Ala Arg Gly Ser Thr Ala
    50              55              60

Glu Gly Lys Asp Thr Gly Phe Tyr Gly His Ala Phe Tyr Ile Glu Arg
65              70              75              80

Lys Asn Gly Gly Gly Ser Lys Asn Asn Ser Ser Gly Ala Gly Asn Ser
            85              90              95

Lys Asp Trp Gly Gly Asn Gly His Gly Asn His Arg Asn Asn Ala Ser
            100             105             110

Asp Leu Asn Lys Pro Asp Gly Asn Asn Gly Asn Asn Gln Asn Asn Gly
            115             120             125

Ser Asn Gln Asp Asn Asn Ser Asp Val Asn Ala Pro Asn Asn Pro Gly
            130             135             140

Arg Asn Tyr Asp Gly Ser Thr Ala Gly Ser Thr Ala Val Ile Asp Ala
145             150             155             160

Gly Ala Gly Asn Asp Thr Ile Asn Gly Gly Tyr Gly Asp Asp Thr Leu
                165             170             175

Ile Gly Gly Lys Gly Asn Asp Ile Leu Lys Gly Ser Tyr Gly Ala Asp
            180             185             190

Thr Tyr Ile Phe Ser Lys Gly His Gly Gln Asp Ile Val Tyr Glu Asp
            195             200             205

Thr Asn Asn Asp Asn Arg Ala Arg Asp Ile Asp Thr Leu Lys Gly Ser
            210             215             220

Thr Ala Gly Ser Thr Ala Phe Leu Asn Lys Leu Leu Ser Thr Ala Lys
225             230             235             240

Glu Arg Asn Arg Trp Glu Glu Pro Gly Gln Lys Leu Tyr Asn Val Glu
                245             250             255

Ala Thr Ser Tyr Ala Gly Ser Thr Ala Phe Leu Asn Lys Leu Leu Ser
            260             265             270
```

70

```
Thr Ala Lys Glu Arg Asn Arg Trp Glu Glu Pro Gly Gln Lys Leu Tyr
        275                 280             285

Asn Val Glu Ala Thr Ser Tyr Ala Gly Ser Thr Ala Phe Leu Asn Lys
        290                 295             300

Leu Leu Ser Thr Ala Lys Glu Arg Asn Arg Trp Glu Glu Pro Gly Gln
305                 310                 315                 320

Lys Leu Tyr Asn Val Glu Ala Thr Ser Tyr Ala Gly Ser Thr Ala Phe
                325                 330             335

Leu Asn Lys Leu Leu Ser Thr Ala Lys Glu Arg Asn Arg Trp Glu Glu
        340                 345                 350

Pro Gly Gln Lys Leu Tyr Asn Val Glu Ala Thr Ser Tyr Ala Gly Ser
        355                 360                 365

Thr Ala Phe Leu Asn Lys Leu Leu Ser Thr Ala Lys Glu Arg Asn Arg
        370                 375                 380

Trp Glu Glu Pro Gly Gln Lys Leu Tyr Asn Val Glu Ala Thr Ser Tyr
385                 390                 395                 400

Ala Gly Ser Thr Ala Phe Leu Asn Lys Leu Leu Ser Thr Ala Lys Glu
                405                 410                 415

Arg Asn Arg Trp Glu Glu Pro Gly Gln Lys Leu Tyr Asn Val Glu Ala
            420                 425                 430

Thr Ser Tyr Ala Gly Ser Thr Ala
            435                 440


<210>  92
<211>  442
<212>  PRT
<213>  Artificial Sequence

<220>
<223>  Recombinant ApxIV toxin with a HindIII site

<400>  92

Val Ile Asp Ala Gly Ala Gly Asn Asp Thr Val Asn Gly Gly Asn Gly
1               5                   10                  15


Asp Asp Thr Leu Ile Gly Gly Lys Gly Asn Asp Ile Leu Arg Gly Gly
            20                  25                  30
```

71

```
Tyr Gly Ala Asp Thr Tyr Ile Phe Ser Lys Gly His Gly Gln Asp Ile
        35                  40                  45

Val Tyr Glu Asp Thr Asn Asn Asp Asn Arg Ala Arg Gly Ser Thr Ala
        50                  55                  60

Glu Gly Lys Asp Thr Gly Phe Tyr Gly His Ala Phe Tyr Ile Glu Arg
65                  70                  75                  80

Lys Asn Gly Gly Gly Ser Lys Asn Asn Ser Ser Gly Ala Gly Asn Ser
                85                  90                  95

Lys Asp Trp Gly Gly Asn Gly His Gly Asn His Arg Asn Asn Ala Ser
            100                 105                 110

Asp Leu Asn Lys Pro Asp Gly Asn Asn Gly Asn Asn Gln Asn Asn Gly
        115                 120                 125

Ser Asn Gln Asp Asn Asn Ser Asp Val Asn Ala Pro Asn Asn Pro Gly
    130                 135                 140

Arg Asn Tyr Asp Gly Ser Thr Ala Gly Ser Thr Ala Val Ile Asp Ala
145                 150                 155                 160

Gly Ala Gly Asn Asp Thr Ile Asn Gly Gly Tyr Gly Asp Asp Thr Leu
                165                 170                 175

Ile Gly Gly Lys Gly Asn Asp Ile Leu Lys Gly Ser Tyr Gly Ala Asp
            180                 185                 190

Thr Tyr Ile Phe Ser Lys Gly His Gly Gln Asp Ile Val Tyr Glu Asp
    195                 200                 205

Thr Asn Asn Asp Asn Arg Ala Arg Asp Ile Asp Thr Leu Lys Gly Ser
    210                 215                 220

Thr Ala Lys Leu Gly Ser Thr Ala Phe Leu Asn Lys Leu Leu Ser Thr
225                 230                 235                 240

Ala Lys Glu Arg Asn Arg Trp Glu Glu Pro Gly Gln Lys Leu Tyr Asn
                245                 250                 255

Val Glu Ala Thr Ser Tyr Ala Gly Ser Thr Ala Phe Leu Asn Lys Leu
            260                 265                 270

Leu Ser Thr Ala Lys Glu Arg Asn Arg Trp Glu Glu Pro Gly Gln Lys
        275                 280                 285
```

72

Leu Tyr Asn Val Glu Ala Thr Ser Tyr Ala Gly Ser Thr Ala Phe Leu
    290             295             300

Asn Lys Leu Leu Ser Thr Ala Lys Glu Arg Asn Arg Trp Glu Glu Pro
305             310             315             320

Gly Gln Lys Leu Tyr Asn Val Glu Ala Thr Ser Tyr Ala Gly Ser Thr
            325             330             335

Ala Phe Leu Asn Lys Leu Leu Ser Thr Ala Lys Glu Arg Asn Arg Trp
            340             345             350

Glu Glu Pro Gly Gln Lys Leu Tyr Asn Val Glu Ala Thr Ser Tyr Ala
        355             360             365

Gly Ser Thr Ala Phe Leu Asn Lys Leu Leu Ser Thr Ala Lys Glu Arg
    370             375             380

Asn Arg Trp Glu Glu Pro Gly Gln Lys Leu Tyr Asn Val Glu Ala Thr
385             390             395             400

Ser Tyr Ala Gly Ser Thr Ala Phe Leu Asn Lys Leu Leu Ser Thr Ala
            405             410             415

Lys Glu Arg Asn Arg Trp Glu Glu Pro Gly Gln Lys Leu Tyr Asn Val
            420             425             430

Glu Ala Thr Ser Tyr Ala Gly Ser Thr Ala
            435             440

<210>  93
<211>  30
<212>  PRT
<213>  Actinobacillus pleuropneumoniae

<400>  93

Val Ile Asp Ala Gly Ala Gly Asn Asp Thr Val Asn Gly Gly Asn Gly
1           5               10              15

Asp Asp Thr Leu Ile Gly Gly Lys Gly Asn Asp Ile Leu Arg
            20              25              30

<210>  94
<211>  4
<212>  PRT
<213>  Actinobacillus pleuropneumoniae

<400>  94

73

```
Gly Tyr Gly Ala
1


<210>  95
<211>  20
<212>  PRT
<213>  Actinobacillus pleuropneumoniae

<400>  95

Ala Gly Ala Gly Asn Asp Thr Val Asn Gly Gly Asn Gly Asp Asp Thr
1               5                   10                  15


Leu Ile Gly Gly
            20


<210>  96
<211>  20
<212>  PRT
<213>  Actinobacillus pleuropneumoniae

<400>  96

Ser Lys Gly His Gly Gln Asp Ile Val Tyr Glu Asp Thr Asn Asn Asp
1               5                   10                  15


Asn Arg Ala Arg
            20


<210>  97
<211>  14
<212>  PRT
<213>  Actinobacillus pleuropneumoniae

<400>  97

Asp Ala Gly Ala Gly Asn Asp Thr Val Asn Gly Gly Asn Gly
1               5                   10


<210>  98
<211>  14
<212>  PRT
<213>  Actinobacillus pleuropneumoniae

<400>  98

Asp Ile Val Tyr Glu Asp Thr Asn Asn Asp Asn Arg Ala Arg
1               5                   10


<210>  99
<211>  20
<212>  PRT
<213>  Actinobacillus pleuropneumoniae

<400>  99
```

```
Ser Lys Gly His Gly Gln Asp Ile Val Tyr Glu Asp Thr Asn Asn Asp
1               5                   10                  15


Asn Arg Ala Arg
            20



<210>   100
<211>   20
<212>   PRT
<213>   Actinobacillus pleuropneumoniae

<400>   100

Lys Asn Gly Gly Gly Ser Lys Asn Asn Ser Ser Gly Ala Gly Asn Ser
1               5                   10                  15


Lys Asp Trp Gly
            20



<210>   101
<211>   20
<212>   PRT
<213>   Actinobacillus pleuropneumoniae

<400>   101

His Arg Asn Asn Ala Ser Asp Leu Asn Lys Pro Asp Gly Asn Asn Gly
1               5                   10                  15


Asn Asn Gln Asn
            20



<210>   102
<211>   20
<212>   PRT
<213>   Actinobacillus pleuropneumoniae

<400>   102

His Arg Asn Asn Ala Ser Asp Leu Asn Lys Pro Asp Gly Asn Asn Gly
1               5                   10                  15


Asn Asn Gln Asn
            20



<210>   103
<211>   14
<212>   PRT
<213>   Actinobacillus pleuropneumoniae

<400>   103

Glu Arg Lys Asn Gly Gly Gly Ser Lys Asn Asn Ser Ser Gly
1               5                   10
```

```
<210>  104
<211>  14
<212>  PRT
<213>  Actinobacillus pleuropneumoniae

<400>  104

Gly Asn Ser Lys Asp Trp Gly Gly Asn Gly His Gly Asn His
1               5                   10


<210>  105
<211>  14
<212>  PRT
<213>  Actinobacillus pleuropneumoniae

<400>  105

Lys Pro Asp Gly Asn Asn Gly Asn Asn Gln Asn Asn Gly Ser
1               5                   10


<210>  106
<211>  14
<212>  PRT
<213>  Actinobacillus pleuropneumoniae

<400>  106

Asp Asn Asn Ser Asp Val Asn Ala Pro Asn Asn Pro Gly Arg
1               5                   10


<210>  107
<211>  20
<212>  PRT
<213>  Actinobacillus pleuropneumoniae

<400>  107

Asn Gly Gly Gly Ser Lys Asn Asn Ser Ser Gly Ala Gly Asn Ser Lys
1               5                   10                  15


Asp Trp Gly Gly
            20


<210>  108
<211>  20
<212>  PRT
<213>  Actinobacillus pleuropneumoniae

<400>  108

Asn Gln Asn Asn Gly Ser Asn Gln Asp Asn Asn Ser Asp Val Asn Ala
1               5                   10                  15


Pro Asn Asn Pro
            20
```

```
<210>   109
<211>   30
<212>   PRT
<213>   Actinobacillus pleuropneumoniae

<400>   109

Val Ile Asp Ala Gly Ala Gly Asn Asp Thr Ile Asn Gly Gly Tyr Gly
1               5                   10                  15


Asp Asp Thr Leu Ile Gly Gly Lys Gly Asn Asp Ile Leu Lys
            20                  25                  30



<210>   110
<211>   4
<212>   PRT
<213>   Actinobacillus pleuropneumoniae

<400>   110

Ser Tyr Gly Ala
1



<210>   111
<211>   19
<212>   PRT
<213>   Actinobacillus pleuropneumoniae

<400>   111

Gly His Gly Gln Asp Ile Val Tyr Glu Asp Thr Asn Asn Asp Asn Arg
1               5                   10                  15


Ala Arg Asp



<210>   112
<211>   20
<212>   PRT
<213>   Actinobacillus pleuropneumoniae

<400>   112

Asp Ala Gly Ala Gly Asn Asp Thr Ile Asn Gly Gly Tyr Gly Asp Asp
1               5                   10                  15


Thr Leu Ile Gly
            20



<210>   113
<211>   20
<212>   PRT
<213>   Actinobacillus pleuropneumoniae
```

<400> 113

Ser Lys Gly His Gly Gln Asp Ile Val Tyr Glu Asp Thr Asn Asn Asp
1               5                   10                  15

Asn Arg Ala Arg
            20

<210> 114
<211> 14
<212> PRT
<213> Actinobacillus pleuropneumoniae

<400> 114

Asp Ala Gly Ala Gly Asn Asp Thr Ile Asn Gly Gly Tyr Gly
1               5                   10

<210> 115
<211> 14
<212> PRT
<213> Actinobacillus pleuropneumoniae

<400> 115

Asp Ile Val Tyr Glu Asp Thr Asn Asn Asp Asn Arg Ala Arg
1               5                   10

<210> 116
<211> 20
<212> PRT
<213> Actinobacillus pleuropneumoniae

<400> 116

Asn Gly Gly Tyr Gly Asp Asp Thr Leu Ile Gly Gly Lys Gly Asn Asp
1               5                   10                  15

Ile Leu Lys Gly
            20

<210> 117
<211> 20
<212> PRT
<213> Actinobacillus pleuropneumoniae

<400> 117

Ser Lys Gly His Gly Gln Asp Ile Val Tyr Glu Asp Thr Asn Asn Asp
1               5                   10                  15

Asn Arg Ala Arg
            20

**Description**

**BACKGROUND OF THE INVENTION**

**1. FIELD OF THE INVENTION**

The present invention relates to preparation and application of recombinant proteins of *Actinobacillus pleuropneumoniae*, particularly to using recombinant Apx toxins to prevent *A. pleuropneumoniae* infection in pigs.

**2. DESCRIPTION OF THE PRIOR ART**

Porcine pleuropneumonia is a highly infectious respiratory disease in pigs caused by *Actinobacillus pleuropneumoniae* (App.). Clinical symptoms of the disease include fever, cough, vomiting, fever, labored breathing, and depression. Infection of the disease can lead to both acute pneumonia with rapid death and chronic infection resulting in asymptomatic carriers. Pigs of all ages are susceptible to porcine pleuropneumonia, in which young pigs less than 6 months have highest morbidity and mortality rates.

*A. pleuropneumoniae* is a gram-negative rod that has two biotypes. Biotype 1 requires β-nicotinamide adenine dinucleotide (β-NAD), whereas biotype 2 is β-NAD independent. In addition, based on differences in capsular polysaccharides, 15 serotypes of A. *pleuropneumoniae* have been recognized, and all the 15 serotypes are capable of causing disease. Several virulence factors including capsule, lipopolysaccharides (LPS), outer membrane proteins (OMP) and, most importantly, the Apx toxins are known.

*A. pleuropneumoniae* RTX (Apx) toxins are members of the repeats in structural toxin (RTX) family. The 15 serotypes of *A. pleuropneumoniae* produce a total of four Apx toxins, and each serotype is able to produce a maximum of three toxins. The ApxI toxin is produced by serotypes 1, 5, 9, 10, 11, and 14. The ApxII toxin is present in all serotypes but serotypes 10 and 14. The ApxIII toxin is produced by serotypes 2, 3, 4, 6, 8, and 15, and the ApxIV toxin is produced by all serotypes but is expressed solely during infection.

Porcine pleuropneumonia causes great economic losses in swine industry worldwide. Treatment of *A. pleuropneumoniae* infection involves using antibiotic such as amoxicillin, ampicillin, ceftiofur, enrofloxacin, tiamulin, penicillin, and penicillin/streptomycin. However, due to growing problems of antibiotic resistance and increasing consumer demand for food safety, preventing *A. pleuropneumoniae* infection by vaccination has become important. Most commercial vaccines against *A. pleuropneumoniae* infection consist of whole cell bacterins. These vaccines reduce mortality but do not prevent initial infection and the development of the carrier state. Therefore, it is important to develop a more effective vaccine against *A. pleuropneumoniae* infection.

**SUMMARY OF THE INVENTION**

In one aspect, the present invention relates to a recombinant Apx toxin (re-Apx), which is represented by Formula (I):

$$(A)_m\text{-}(C3d \text{ fragment})_n \qquad (I)$$

wherein

each A is an individual epitope of an Apx toxin;
each C3d fragment is an individual unit of the amino acid sequence of complement C3d and is independently selected from the group consisting of SEQ ID NOs: 22, 23, 24, and 25;
m is an integer from 1 to about 30; and
n is an integer from 0 to about 10.

In another aspect, the present invention relates to a nucleotide sequence encoding the recombinant Apx toxin (re-Apx).

In a further aspect, the present invention relates to an immunogenic composition against porcine pleuropneumonia. The immunogenic composition against porcine pleuropneumonia comprises the recombinant Apx toxin (re-Apx) and a pharmaceutically acceptable vehicle.

In another aspect, the present invention relates to a method of protecting an animal against porcine pleuropneumonia. The method comprises administering an effective amount of the immunogenic composition above to the animal to increase immunity against porcine pleuropneumonia in the animal.

In a further aspect, the present invention relates to an anti-Apx toxin antibody. The antibody is prepared or derived from the recombinant Apx toxin (re-Apx) of the present invention.

In yet another aspect, the present invention relates to a test kit for porcine pleuropneumonia. The test kit is used to detect Apx toxin or anti-Apx toxin antibodies in a sample.

The immunogenic composition against porcine pleuropneumonia of the present invention has the following advantages.

The immunogenic composition against porcine pleuropneumonia provided by the present invention comprises a recombinant Apx toxin (re-Apx). The recombinant Apx toxin (re-Apx) comprises at least one epitope of an Apx toxin and partial-length of the amino acid sequence of complement component 3d (C3d). The recombinant Apx toxin (re-Apx) is much shorter than the full-length of an Apx toxin, so the recombinant toxin can be relatively easily expressed in a biological expression system with higher yields and lower costs.

Furthermore, because the recombinant Apx toxin (re-Apx) of the present invention comprises partial-length of the amino acid sequence of C3d, the recombinant protein can increase specific immune response. Experimental data shows that the immunogenic composition against porcine pleuropneumonia comprising the recombinant Apx toxin (re-Apx) of the present invention improves immunity and efficacy against porcine pleuropneumonia in animals.

The present invention is described in more detail in the following illustrative examples. Although the examples may represent only selected embodiments of the invention, it should be understood that the following examples are illustrative and not limiting.

## BRIEF DESCRIPTION OF THE DRAWINGS

The accompanying drawings illustrate one or more embodiments of the invention and together with the written description, serve to explain the principles of the invention. Wherever possible, the same reference numbers are used throughout the drawings to refer to the same or like elements of an embodiment, and wherein:

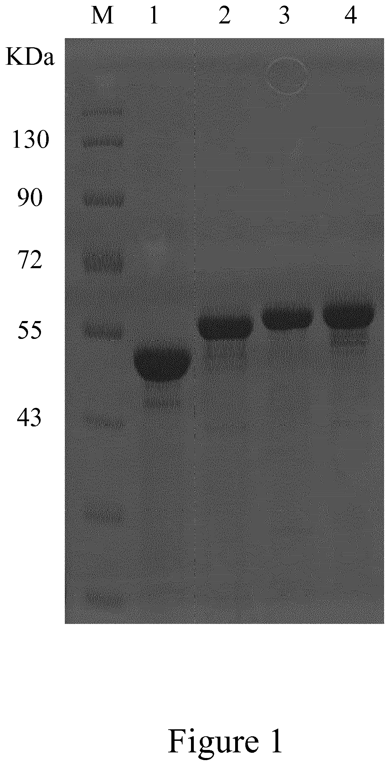

Figure 1 shows a sodium dodecyl sulfate polyacrylamide gel electrophoresis (SDS-PAGE) (10%) analysis of purified recombinant Apx toxins (re-Apx) according to Examples 1-4 of the present invention; M: protein markers; lane 1: recombinant ApxI toxin (re-ApxI); lane 2: recombinant ApxII toxin (re-ApxII); lane 3: recombinant ApxIII toxin (re-ApxIII); lane 4: recombinant ApxIV toxin (re-ApxIV).

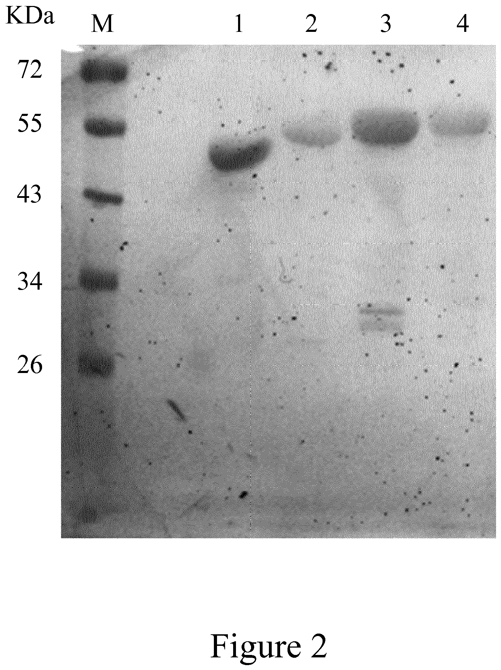

Figure 2 shows western blots of recombinant Apx toxins (re-Apx) according to Examples 1-4 of the present invention; M: protein markers; lane 1: recombinant ApxI toxin (re-ApxI); lane 2: recombinant ApxII toxin (re-ApxII); lane 3: recombinant ApxIII toxin (re-ApxIII); lane 4: recombinant ApxIV toxin (re-ApxIV). The primary antibody used in this analysis was alkaline phosphatase-conjugated mouse anti-His Tag antibody (Invitrogen, MA, USA).

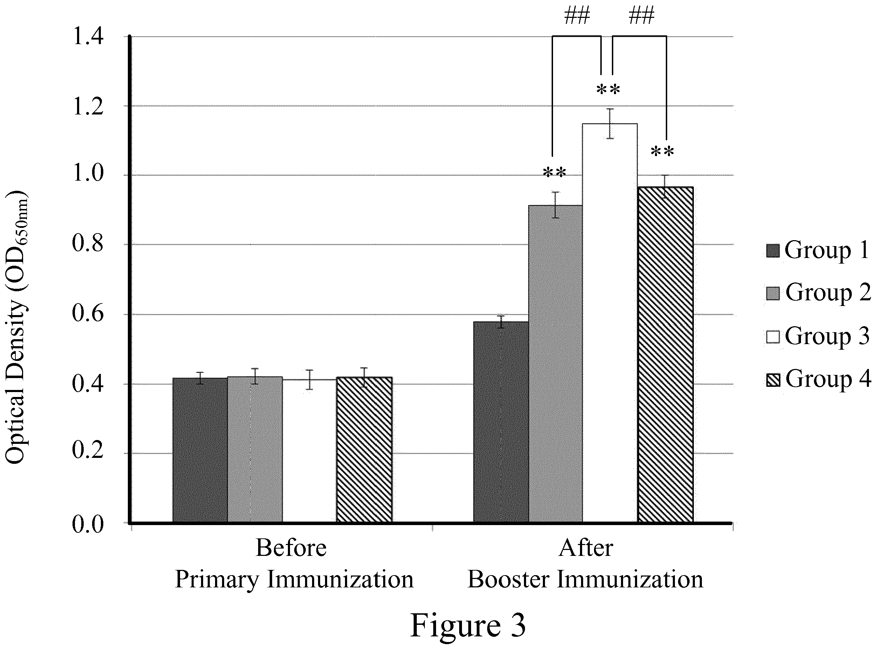

Figure 3 shows results of enzyme-linked immunosorbent assay (ELISA) of antibodies against recombinant ApxII toxin (re-ApxII) according to Example 9 of the present invention; Group 1 is the negative control group (PBS group); Group 2 is the multivalent *A. pleuropneumoniae* bacterin containing serotypes 1, 2, 5 obtained in Example 6 (App 1, 2, 5 group); Group 3 is the multivalent vaccine containing *A. pleuropneumoniae* bacterin and recombinant Apx Toxin obtained in Example 6 (App 1, 2, 5 + re-ApxI~III group); Group 4 is a commercial *A. pleuropneumoniae* bacterin containing serotypes 1, 2, 3, 4, 5, and 7 (App 1, 2, 3, 4, 5, 7 group). ** indicates there is a significant difference between a group and the negative control group ($p<0.01$); ## indicates there is a significant difference between two groups ($p<0.01$).

## DETAILED DESCRIPTION OF THE PREFERRED EMBODIMENT

The present invention provides a recombinant Apx toxin (re-Apx). The recombinant toxin has at least one epitope of an Apx toxin to induce animals to produce anti-Apx toxin antibody. The recombinant Apx toxin (re-Apx) may further have at least one unit of partial- or full-length of the amino acid sequence of complement component 3d (C3d) to increase specific immune response. The recombinant Apx toxin (re-Apx) of the present invention may be represented by Formula (I):

$$(A)_m\text{-}(C3d\ fragment)_n \qquad\qquad (I)$$

wherein

each A is an individual epitope of an Apx toxin;

each C3d fragment is an individual unit of the amino acid sequence of complement C3d;

m is an integer from 1 to about 30; and

n is an integer from 0 to about 10.

In an embodiment, the full-length of the amino acid sequence of the C3d is the full-length of the amino acid sequence of a mouse complement component 3d (mC3d), as shown in SEQ ID NO: 25. In a preferred embodiment, the partial-length of the amino acid sequence of the C3d is the 211th to the 238th amino acids of the mouse C3d (mC3d-p28), which has the sequence of SEQ ID NO: 24. In another embodiment, the full-length of the amino acid sequence of the C3d is the full-length of the amino acid sequence of a pig C3d (pC3d), as shown in SEQ ID NO: 23. In another preferred embodiment, the partial-length of the amino acid sequence of the C3d is the 201st to the 231st amino acids of the pig C3d (pC3d-p31), which has the sequence of SEQ ID NO: 22. The recombinant Apx toxin (re-Apx) of the present invention has 0 to 10 repeated units of the partial- or full-length of the amino acid sequence of C3d, preferably 1 to 10 repeated units, more preferably 4 to 8 repeated units.

In an embodiment, each A is linked to another A with a linker, and each linker is independently selected from Gly-Gly, Gly-Ser, and SEQ ID NOs: 26, 27, 28, 29, 30, 31, 32, 33, 34, 35, and 36.

In an embodiment, each C3d fragment is linked to another C3d fragment with a linker, and each linker is independently selected from Gly-Gly, Gly-Ser, and SEQ ID NOs: 26, 27, 28, 29, 30, 31, 32, 33, 34, 35, and 36.

In an embodiment, an A and a C3d fragment are linked with a linker, and each linker is independently selected from Gly-Gly, Gly-Ser, and SEQ ID NOs: 26, 27, 28, 29, 30, 31, 32, 33, 34, 35, and 36.

In an embodiment, the Apx toxin is ApxI toxin, the recombinant Apx toxin is re-ApxI toxin, and each A is independently selected from the amino acid sequences of SEQ ID NOs: 37, 4, 39, 40, 41, 42, 43, 44, 45, 46, 47, 48, 49, 50, and 51. The number of the epitopes of ApxI toxin is 1 to about 30. The epitopes of ApxI toxin from the N-terminus to the C-terminus of the re-ApxI toxin are not necessarily arranged in ascending or descending order of SEQ ID Numbers. In a preferred embodiment, some of the epitopes of ApxI toxin mentioned above are included in one of five longer epitope fragments of ApxI toxin. The amino acid sequence of each of the five longer epitope fragments of ApxI toxin is SEQ ID NOs: 2, 3, 4, 5, and 6, respectively. In a preferred embodiment, the re-ApxI toxin of the present invention comprises at least two of the longer epitope fragments of ApxI toxin, and the longer epitope fragments of ApxI toxin from the N-terminus to the C-terminus of the re-ApxI toxin are not necessarily arranged in ascending or descending order of SEQ ID Numbers. In a preferred embodiment, the re-ApxI toxin of the present invention comprises the amino acid sequences of SEQ ID NOs: 7 or 8.

In an embodiment, the Apx toxin is ApxII toxin, the recombinant Apx toxin is re-ApxII toxin, and each A is independently selected from the amino acid sequences of SEQ ID NOs: 14, 52, 53, 54, 55, 56, 57, 58, 59, 60, 61, 62, 63, 64, 65, 67, and 68. The number of the epitopes of ApxII toxin is 1 to about 30. The epitopes of ApxII toxin from the N-terminus to the C-terminus of the re-ApxII toxin are not necessarily arranged in ascending or descending order of SEQ ID Numbers. In a preferred embodiment, some of the epitopes of ApxII toxin mentioned above are included in one of five longer epitope fragments of ApxII toxin. The amino acid sequence of each of the five longer epitope fragments of ApxII toxin is SEQ ID NOs: 10, 11, 12, 13, and 14, respectively. In a preferred embodiment, the re-ApxII toxin of the present invention comprises at least two of the longer epitope fragments of ApxII toxin, and the longer epitope fragments of ApxII toxin from the N-terminus to the C-terminus of the re-ApxII toxin are not necessarily arranged in ascending or descending order of SEQ ID Numbers. In a preferred embodiment, the re-ApxII toxin of the present invention comprises the amino acid sequences of SEQ ID NOs: 15 or 16.

In an embodiment, the Apx toxin is ApxIII toxin, the recombinant Apx toxin is re-ApxIII toxin, and each A is independently selected from the amino acid sequences of SEQ ID NOs: 69, 70, 71, 72, 73, 74, 75, 76, 77, 78, 79, 80, 81, 82, 83, 84, 85, 86, 87, and 88. The number of the epitopes of ApxIII toxin is 1 to about 30. The epitopes of ApxIII toxin from the N-terminus to the C-terminus of the re-ApxIII toxin are not necessarily arranged in ascending or descending order of SEQ ID Numbers. In a preferred embodiment, some of the epitopes of ApxIII toxin mentioned above are included in one of two longer epitope fragments of ApxIII toxin. The amino acid sequence of each of the two longer epitope fragments of ApxIII toxin is SEQ ID NOs: 18 and 19, respectively. In a preferred embodiment, the re-ApxIII toxin of the present invention comprises the two longer epitope fragments of ApxIII toxin, and the epitopes of ApxIII toxin from the N-terminus to the C-terminus of the re-ApxIII toxin are not necessarily arranged in ascending or descending order of SEQ ID Numbers. In a preferred embodiment, the re-ApxIII toxin of the present invention comprises the amino acid sequences of SEQ ID NOs: 20 or 21.

In an embodiment, the Apx toxin is ApxIV toxin, the recombinant Apx toxin is re-ApxIV toxin, and each A is independently selected from the amino acid sequences of SEQ ID NOs: 93, 94, 95, 96, 97, 98, 99, 100, 101, 102, 103, 104, 105, 106, 107, 108, 109, 110, 111, 112, 113, 114, 115, 116, and 117. The number of the epitopes of ApxIV toxin is 1 to about 30. The epitopes of ApxIV toxin from the N-terminus to the C-terminus of the re-ApxIV toxin are not necessarily arranged in ascending or descending order of SEQ ID Numbers. In a preferred embodiment, some of the epitopes of ApxIV toxin mentioned above are included in one of three longer epitope fragments of ApxIV toxin. The amino acid sequence of each of the three longer epitope fragments of ApxIV toxin is SEQ ID NOs: 66, 89, and 90, respectively. In a preferred embodiment, the re-ApxIV toxin of the present invention comprises at least two of the longer epitope fragments of ApxIV toxin, and the epitopes of ApxIV toxin from the N-terminus to the C-terminus of the re-ApxIV toxin are not necessarily arranged in ascending or descending order of SEQ ID Numbers. In a preferred embodiment, the re-ApxIV toxin of the present invention comprises the amino acid sequences of SEQ ID NOs: 91 or 92.

In some examples, the recombinant Apx toxin (re-Apx) of the present invention comprises an amino acid sequence having at least about 80%, preferably about 85%, more preferably aobut 90%, even preferably about 91%, about 92%, about 93%, about 94%, about 95%, about 96%, about 97%, about 98%, or about 99% sequence identity to the amino acid sequence represented by Formula (I).

The invention also provides a polynucleotide sequence encoding the recombinant Apx toxin (re-Apx) of the present invention. The recombinant Apx toxin (re-Apx) comprises at least one epitope of an Apx toxin and 0 to 10 repeated units of the partial- or full-length of the amino acid sequence of C3d. The polynucleotide sequence encoding the recombinant Apx toxin (re-Apx) of the present invention is derived from the amino acid sequence of the recombinant Apx toxin (re-Apx) by replacing each amino acid with a three-nucleotide codon, including every degenerate codon (also called synonymous codons). For example, each serine of the amino acid sequence of the recombinant Apx toxin (re-Apx) can be independently encoded by the codons TCT, TCC, TCA, TCG, AGT, or AGC.

In addition, the present invention provides an immunogenic composition against porcine pleuropneumonia. The immunogenic composition of the present invention comprises the recombinant Apx toxin (re-Apx) of the present invention and a pharmaceutically acceptable vehicle. In an embodiment, the recombinant Apx toxin (re-Apx) is at least one of the re-ApxI toxin, re-ApxII toxin, re-ApxIII toxin, and re-ApxIV toxin. In a preferred embodiment, the immunogenic composition against porcine pleuropneumonia comprises the re-ApxI toxin, re-ApxII toxin, and re-ApxIII toxin and a pharmaceutically acceptable vehicle. In a preferred embodiment, the immunogenic composition against porcine pleuropneumonia comprises the re-ApxI toxin, re-ApxII toxin, re-ApxIII toxin, and re-ApxIV toxin and a pharmaceutically acceptable vehicle.

In an embodiment, the immunogenic composition against porcine pleuropneumonia of the present invention further comprises at least one serotype of *A. pleuropneumoniae.* The serotype of *A. pleuropneumoniae* includes, but is not limited to, serotypes 1, 2, 3, 4, 5, 6, 7, 8, 9, 10, 11, 12, 13, 14, and 15 of *A. pleuropneumoniae.* In a preferred embodiment, the immunogenic composition against porcine pleuropneumonia further comprises serotypes 1, 2, and 5 of *A. pleuropneumoniae.*

In an embodiment, the immunogenic composition against porcine pleuropneumonia of the present invention further comprises at least one other pathogen antigen. The pathogen antigens include, but are not limited to, antigen of porcine circovirus type 2 (PCV2), antigen of Swine influenza virus (SIV), antigen of porcine reproductive and respiratory syndrome virus (PRRSV), antigen of mycoplasma, antigen of porcine parvovirus (PPV), antigen of erysipelas, antigen of *Bordetella bronchiseptica,* antigen of *Pasteurella multocida,* and antigen of pseudorabies (Aujeszky's disease) virus.

The immunogenic composition against porcine pleuropneumonia of the present invention may further comprise at least one pharmaceutically acceptable vehicles including, but not limited to, solvent, emulsifier, suspending agent, decomposer, binding agent, excipient, stabilizing agent, chelating agent, diluent, gelling agent, preservative, lubricant, surfactant, adjuvant, biological carriers, or other suitable vehicles.

The pharmaceutically acceptable vehicle comprises at least one of the following reagents: solvent, emulsifier, suspending agent, decomposer, binding agent, excipient, stabilizing agent, chelating agent, diluent, gelling agent, preservative, lubricant, surfactant, adjuvant, and other suitable vehicles.

The excipient may be pharmaceutically acceptable organic or inorganic carrier substances suitable for parenteral, enteral or intranasal application which do not deleteriously react with the active compounds and are not deleterious to the recipient thereof. Suitable excipients include, but are not limited to, water, salt solutions, vegetable oils, polyethylene glycols, gelatin, lactose, amylose, magnesium stearate, talc, silicic acid, viscous paraffin, fatty acid monoglycerides and diglycerides, petroethral fatty acid esters, hydroxymethyl-cellulose, polyvinylpyrrolidone, etc.

The pharmaceutically acceptable adjuvant includes, but not limited to, aluminum hydroxide, potassium alum, Freund's Complete Adjuvant, Freund's Incomplete Adjuvant, oil adjuvant (such as mineral oil, plant oil, animal oil etc.), aqueous adjuvant, biphasic emulsification adjuvant (such as water-in-oil-in-water emulsion adjuvant), biological adjuvant (such as CpG oligodeoxynucleotide and toxoid), etc. In an embodiment, the adjuvant is aluminum hydroxide.

Furthermore, the invention provides a method of protecting an animal against porcine pleuropneumonia, comprising administering an effective amount of the immunogenic composition of the present invention to the animal to increase immunity against porcine pleuropneumonia in the animal, so that the surviving rate after infection of *A. pleuropneumoniae* can be improved.

The present invention also provides an anti-Apx toxin antibody prepared or derived from the recombinant Apx toxin (re-Apx) of the present invention. The antibody includes, but is not limited to, monoclonal antibodies, polyclonal antibodies, and genetically engineered antibodies. In an embodiment, the antibody is a polyclonal antibody obtained via injecting an animal with the recombinant Apx toxin (re-Apx).

The present invention further provides a test kit for porcine pleuropneumonia. The test kit is used to detect Apx toxin or anti-Apx toxin antibodies in a test sample. The test kit includes, but is not limited to: (1) an antigen of the recombinant Apx toxin (re-Apx), in one preferred embodiment, the antigen is deposited on an antigen plate, and/or (2) a monoclonal or polyclonal antibody prepared or derived from the recombinant Apx toxin (re-Apx).

The types of the test kit include, but are not limited to, enzyme-linked immunosorbent assay kit (ELISA), microchip kit, immunofluorescent assay (IFA) kit, and other test kits derived from the recombinant Apx toxin (re-Apx). In an embodiment, the test kit comprises at least an antigen plate comprising the recombinant Apx toxin (re-Apx), and the test kit can be used to detect anti-Apx toxin antibodies in a test sample.

As used herein, the singular forms "a", "an", and "the" include plural referents unless the context clearly dictates otherwise. Thus, for example, reference to "a component" includes a plurality of such components and equivalents thereof known to those skilled in the art.

As used herein, "around", "about" or "approximately" shall generally mean within 20 percent, preferably within 10 percent, and more preferably within 5 percent of a given value or range. Numerical quantities given herein are approximate, meaning that the term "around", "about" or "approximately" can be inferred if not expressly stated.

The meaning of the technical and scientific terms as described herein can be clearly understood by a person of ordinary skill in the art.

The present invention is described in more detail in the following illustrative examples. Although the examples may represent only selected embodiments of the invention, it should be understood that the following examples are illustrative and not limiting.

**Example 1**

**Construction of re-ApxI toxin**

Five epitope fragments were selected from the full-length amino acid sequence of Apx I toxin (SEQ ID NO: 1). Sequences of the five epitope fragments are listed below.

Epitope fragment ApxI-1: ELAGITRKGADAKSGK (SEQ ID NO: 2);
Epitope fragment ApxI-2: PAGVGAAA (SEQ ID NO: 3);
Epitope fragment ApxI-3: DILYGSDGTNLFDGGVGNDKIYGG (SEQ ID NO: 4);
Epitope fragment ApxI-4: EHGQVLVGAGGPLAYSNSPNSIPNAF (SEQ ID NO: 5);
Epitope fragment ApxI-5:

RAKDELHSVEEIIGSNRKDKFFGSRFTDIFHGAKGDDEIYGNDGHDILYGDDGNDVIH

GGDGNDHLVGGNGNDRLIGGKGNNFLNGGDGDDELLQVFE (SEQ ID NO: 6);

These epitope fragments contain, but are not limited to, the following epitopes:

RKGADAKSGK (SEQ ID NO: 37);
DILYGSDGTNLFDGGVGNDKIYGG (SEQ ID NO: 4);
GGPLAYSNSPNSIPNA (SEQ ID NO: 39);
GAGGPLAYSNSPNS (SEQ ID NO: 40);
LVGAGGPLAYSNSPNSIPNA (SEQ ID NO: 41);
GSNRKD (SEQ ID NO: 42);
GAKGDDEIYGNDGHDILYGDDGNDVIHGGDGNDHLVGGNGNDRLIGG (SEQ ID NO: 43);
NNFLNGGDGDDEL (SEQ ID NO: 44);
GSRFTDIFHGAKGDDEIYGN (SEQ ID NO: 45);
DVIHGGDGNDHLVGGNGNDR (SEQ ID NO: 46);
IGGKGNNFLNGGDGDDELQV (SEQ ID NO: 47);
HGAKGDDEIYGNDG (SEQ ID NO: 48);
GGKGNNFLNGGDGD (SEQ ID NO: 49);
DGHDILYGDDGNDVIHGGDG (SEQ ID NO: 50);
RLIGGKGNNFLNGGDGDDEL (SEQ ID NO: 51).

Epitope fragments ApxI-1, ApxI-2, ApxI-3, ApxI-4, and ApxI-5 were linked from N- to C- terminus, and each epitope fragment was linked to another epitope fragment with at least one linker having the amino acid sequence of SEQ ID NO: 26. Six (6) repeats of pC3d-p31 bioadjuvant (SEQ ID NO: 22) were added to the C-terminus of epitope fragment ApxI-5, in which each pC3d-p31 bioadjuvant was linked to another pC3d-p31 bioadjuvant with at least one linker (SEQ ID NO: 26). The designed recombinant ApxI toxin (re-ApxI) has the amino acid sequence of SEQ ID NO: 7. The amino acid sequence of re-ApxI toxin was synthesized by a peptide synthesizer. Alternatively, a polynucleotide sequence encoding the amino acid sequence of re-ApxI toxin was synthesized and cloned in an expression vector, the expression vector was transfected into a host cell, and then the amino acid sequence of re-ApxI toxin was expressed by the host cell and purified.

Alternatively, a DNA sequence encoding the amino acid sequence of re-ApxI toxin was constructed by molecular cloning. Then, the polynucleotide sequence was inserted in an expression vector through restriction enzyme sites, and the expression vector was transfected into a host cell. Finally, the amino acid sequence of re-ApxI toxin was expressed by the host cell and purified. In this Example, a DNA sequence encoding the epitope fragments of ApxI toxin and a DNA sequence encoding pC3d-p31 bioadjuvant were linked with a HindIII restriction enzyme site, and the amino acid sequence of re-ApxI toxin obtained by the cloning is SEQ ID NO: 8. The obtained re-ApxI toxin was purified by nickel affinity chromatography and ion exchange chromatography. Then the purified re-ApxI toxin was confirmed by sodium dodecyl sulfate polyacrylamide gel electrophoresis (SDS-PAGE) and western blot, and the results are shown in Figure 1 (lane 1) and Figure 2 (lane 2), respectively. The results indicate that the re-ApxI toxin has the expected molecular weight of about 46kDa.

### Example 2

### Construction of re-ApxII toxin

Five epitope fragments were selected from the full-length amino acid sequence of ApxII toxin (SEQ ID NO: 9). Sequences of the five epitope fragments are listed below.

Epitope fragment ApxII-1:

GNGVDTIDGNDGDDHLFGGAGDDVIDGGNGNNFLVGGTGNDIISGGKDNDIYVHKT

GDGNDSITDSGGQDKL (SEQ ID NO: 10);

Epitope fragment ApxII-2: KVGNDYNTSKDRQNV (SEQ ID NO: 11);
Epitope fragment ApxII-3: LTPGEENRERIQEGKNSYITKLHIQRVDSWTVTDGDASSSV (SEQ ID NO: 12);
Epitope fragment ApxII-4: ILYIPQGYDSGQGNGVQDLV (SEQ ID NO: 13);
Epitope fragment ApxII-5: ATHPTNVGNREEKIEYRREDDRFH (SEQ ID NO: 14).

These epitope fragments contain, but are not limited to, the following epitopes:

GTGNDIISGGKDNDI (SEQ ID NO: 52);
HKTGDGNDSITDSGGQDKL (SEQ ID NO: 53);
FGGAGDDVIDGGNGNNFLVG (SEQ ID NO: 54);
YVHKTGDGNDSITDSGGQDK (SEQ ID NO: 55);
GGAGDDVIDGGNGN (SEQ ID NO: 56);
GGTGNDIISGGKDN (SEQ ID NO: 57);
KTGDGNDSITDSGG (SEQ ID NO: 58);
AGDDVIDGGNGNNFLVGGTG (SEQ ID NO: 59);
DIYVHKTGDGNDSITDSGGQ (SEQ ID NO: 60);
LTPGEENRERIQEGKNS (SEQ ID NO: 61);
WTVTDGDASSSV (SEQ ID NO: 62);
TPGEENRERIQEGKNSYITK (SEQ ID NO: 63);
LFRTPLLTPGEENR (SEQ ID NO: 64);
QGYDSGQGNGVQ (SEQ ID NO: 65);
ATHPTNVGNREEKIEYRREDDRFH (SEQ ID NO: 14);
NREEKIEYRREDDR (SEQ ID NO: 67);
PTNVGNREEKIEYRREDDRF (SEQ ID NO: 68).

Epitope fragments ApxII-1, ApxII-2, ApxII-3, ApxII-4, and ApxII-5 were linked from N-to C- terminus, and each epitope fragment was linked to another epitope fragment with at least one linker having the amino acid sequence of SEQ ID NO: 26. Six (6) repeats of pC3d-p31 bioadjuvant (SEQ ID NO: 22) were added to the C-terminus of epitope fragment ApxII-5, in which each pC3d-p31 bioadjuvant was linked to another pC3d-p31 bioadjuvant with at least one linker (SEQ ID NO: 26). The designed recombinant ApxII toxin (re-ApxII) has the amino acid sequence of SEQ ID NO: 15. The amino acid sequence of re-ApxII toxin was synthesized by a peptide synthesizer. Alternatively, a polynucleotide sequence encoding the amino acid sequence of re-ApxII toxin was synthesized and cloned in an expression vector, the expression vector was transfected into a host cell, and then the amino acid sequence of re-ApxII toxin was expressed by the host cell and purified.

Alternatively, a DNA sequence encoding the amino acid sequence of re-ApxII toxin was constructed by molecular cloning. Then, the polynucleotide sequence was inserted in an expression vector through restriction enzyme sites, and the expression vector was transfected into a host cell. Finally, the amino acid sequence of re-ApxII toxin was expressed by the host cell and purified. In this Example, a DNA sequence encoding the epitope fragments of ApxII toxin and a DNA sequence encoding pC3d-p31 bioadjuvant were linked with a *Hind*III restriction enzyme site, and the amino acid sequence of re-ApxII toxin obtained by the cloning is SEQ ID NO: 16. The obtained re-ApxII toxin was purified by nickel affinity chromatography and ion exchange chromatography. Then the purified re-ApxII toxin was confirmed by sodium dodecyl sulfate polyacrylamide gel electrophoresis (SDS-PAGE) and western blot, and the results are shown in Figure 1 (lane 2) and Figure 2 (lane 3), respectively. The results indicate that the re-ApxII toxin has the expected molecular weight of about 47kDa.

**Example 3**

**Construction of re-ApxIII toxin**

Two epitope fragments were selected from the full-length amino acid sequence of Apx III toxin (SEQ ID NO: 17). Sequences of the two epitope fragments are listed below.

Epitope fragment ApxIII-1:

ADGDDLLNGNDGDDILYGDKGNDELRGDNGNDQLYGGEGNDKLLGGNGNNYLSG
GDGNDELQVLGNGFNVLRAGKGDDKLYGSSGSDLLDGGEGNDYLEGGDGSDFYVY
RSTSGNHTIYDQGKSSDL (SEQ ID NO: 18);

Epitope fragment ApxIII-2:

GELAGITGKGDKLSSGKAYVDYFQEGKLLEKKPDDFSKVVFDPTKGEIDISNSQTSTL
LKFVTPLLTPGTESRERTQTGK (SEQ ID NO: 19).

These epitope fragments contain, but are not limited to, the following epitopes:

GADGDDLLNGNDGDDILYGDKGNDELRGDNGNDQLYGGEGNDKLLGGNGNNYLS
GGDGNDEL (SEQ ID NO: 69);

AGKGDDKLYGSSGSDLLDGGEGNDYLEGGDGSD (SEQ ID NO: 70);
STSGNHTIYDQGKSSD (SEQ ID NO: 71);
GDKGNDELRGDNGNDQLYGG (SEQ ID NO: 72);
LLGGNGNNYLSGGDGNDELQ (SEQ ID NO: 73);
GDKGNDELRGDNGN (SEQ ID NO: 74);
GGNGNNYLSGGDGN (SEQ ID NO: 75);
DGDDILYGDKGNDELRGDNG (SEQ ID NO: 76);
LLGGNGNNYLSGGDGNDELQ (SEQ ID NO: 77);
NGFNVLRAGKGDDKLYGSSG (SEQ ID NO: 78);

GITGKGDKLSSGKA (SEQ ID NO: 79);
LLEKKPDDF (SEQ ID NO: 80);
FDPTKGEIDISNSQT (SEQ ID NO: 81);
GITGKGDKLSSGKAYVDYFQ (SEQ ID NO: 82);
VFDPTKGEIDISNSQTSTLL (SEQ ID NO: 83);
KGDKLSSGKAYVDY (SEQ ID NO: 84);
EKKPDDFSKVVFDP (SEQ ID NO: 85);
FVTPLLTPGTESRE (SEQ ID NO: 86);
ELAGITGKGDKLSSGKAYVD (SEQ ID NO: 87);
TLLKFVTPLLTPGTESRERT (SEQ ID NO: 88).

Epitope fragments ApxIII-1 and ApxIII-2 were linked from N- to C- terminus, and each epitope fragment was linked to another epitope fragment with at least one linker having the amino acid sequence of SEQ ID NO: 26. Six (6) repeats of pC3d-p31 bioadjuvant (SEQ ID NO: 22) were added to the C-terminus of epitope fragment ApxIII-2, in which each pC3d-p31 bioadjuvant was linked to another pC3d-p31 bioadjuvant with at least one linker (SEQ ID NO: 26). The designed recombinant ApxIII toxin (re-ApxIII) has the amino acid sequence of SEQ ID NO: 20. The amino acid sequence of re-ApxIII toxin was synthesized by a peptide synthesizer. Alternatively, a polynucleotide sequence encoding the amino acid sequence of re-ApxIII toxin was synthesized and cloned in an expression vector, the expression vector was transfected into a host cell, and then the amino acid sequence of re-ApxIII toxin was expressed by the host cell and purified.

Alternatively, a DNA sequence encoding the amino acid sequence of re-ApxIII toxin was constructed by molecular cloning. Then, the polynucleotide sequence was inserted in an expression vector through restriction enzyme sites, and the expression vector was transfected into a host cell. Finally, the amino acid sequence of re-ApxIII toxin was expressed by the host cell and purified. In this Example, a DNA sequence encoding the epitope fragments of ApxIII toxin and a DNA sequence encoding pC3d-p31 bioadjuvant were linked with a *Hind*III restriction enzyme site, and the amino acid sequence of re-ApxIII toxin obtained by the cloning is SEQ ID NO: 21. The obtained re-ApxIII toxin was purified by nickel affinity chromatography and ion exchange chromatography. Then the purified re-ApxIII toxin was confirmed by sodium dodecyl sulfate polyacrylamide gel electrophoresis (SDS-PAGE) and western blot, and the results are shown in Figure 1 (lane 3) and Figure 2 (lane 4), respectively. The results indicate that the re-ApxIII toxin has the expected molecular weight of about 49kDa.

**Example 4**

**Construction of re-ApxIV toxin**

Three epitope fragments were selected from the full-length amino acid sequence of ApxIV toxin (SEQ ID NO: 38). Sequences of the three epitope fragments are listed below.

Epitope fragment ApxIV-1:

VIDAGAGNDTVNGGNGDDTLIGGKGNDILRGGYGADTYIFSKGHGQDIVYEDTNND

NRAR (SEQ ID NO: 66);

Epitope fragment ApxIV-2:

EGKDTGFYGHAFYIERKNGGGSKNNSSGAGNSKDWGGNGHGNHRNNASDLNKPDG

NNGNNQNNGSNQDNNSDVNAPNNPGRNYD (SEQ ID NO: 89);

Epitope fragment ApxIV-3:

VIDAGAGNDTINGGYGDDTLIGGKGNDILKGSYGADTYIFSKGHGQDIVYEDTNNDN

RARDIDTLK (SEQ ID NO: 90).

These epitope fragments contain, but are not limited to, the following epitopes:

VIDAGAGNDTVNGGNGDDTLIGGKGNDILR (SEQ ID NO: 93);
GYGA (SEQ ID NO: 94);
AGAGNDTVNGGNGDDTLIGG (SEQ ID NO: 95);
SKGHGQDIVYEDTNNDNRAR (SEQ ID NO: 96);
DAGAGNDTVNGGNG (SEQ ID NO: 97);
DIVYEDTNNDNRAR (SEQ ID NO: 98);
SKGHGQDIVYEDTNNDNRAR (SEQ ID NO: 99);
KNGGGSKNNSSGAGNSKDWG (SEQ ID NO: 100);
HRNNASDLNKPDGNNGNNQN (SEQ ID NO: 101);
GSNQDNNSDVNAPNNPGRNY (SEQ ID NO: 102);
ERKNGGGSKNNSSG (SEQ ID NO: 103);
GNSKDWGGNGHGNH (SEQ ID NO: 104);
KPDGNNGNNQNNGS (SEQ ID NO: 105);
DNNSDVNAPNNPGR (SEQ ID NO: 106);
NGGGSKNNSSGAGNSKDWGG (SEQ ID NO: 107);
NQNNGSNQDNNSDVNAPNNP (SEQ ID NO: 108);
VIDAGAGNDTINGGYGDDTLIGGKGNDILK (SEQ ID NO: 109);
SYGA (SEQ ID NO: 110);
GHGQDIVYEDTNNDNRARD (SEQ ID NO: 111);
DAGAGNDTINGGYGDDTLIG (SEQ ID NO: 112);
SKGHGQDIVYEDTNNDNRAR (SEQ ID NO: 113);
DAGAGNDTINGGYG (SEQ ID NO: 114);
DIVYEDTNNDNRAR (SEQ ID NO: 115);
NGGYGDDTLIGGKGNDILKG (SEQ ID NO: 116);
SKGHGQDIVYEDTNNDNRAR (SEQ ID NO: 117).

Epitope fragments ApxIV-1, ApxIV-2, and ApxIV-3 were linked from N- to C- terminus, and each epitope fragment was linked to another epitope fragment with at least one linker having the amino acid sequence of SEQ ID NO: 26. Six (6) repeats of pC3d-p31 bioadjuvant (SEQ ID NO: 22) were added to the C-terminus of epitope fragment ApxIV-3, in which each pC3d-p31 bioadjuvant was linked to another pC3d-p31 bioadjuvant with at least one linker (SEQ ID NO: 26). The designed recombinant ApxIV toxin (re-ApxIV) has the amino acid sequence of SEQ ID NO: 91. The amino acid sequence of re-ApxIV toxin was synthesized by a peptide synthesizer. Alternatively, a polynucleotide sequence encoding the amino acid sequence of re-ApxIV toxin was synthesized and cloned in an expression vector, the expression vector was transfected into a host cell, and then the amino acid sequence of re-ApxIV toxin was expressed by the host cell and purified.

Alternatively, a DNA sequence encoding the amino acid sequence of re-ApxIV toxin was constructed by molecular cloning. Then, the polynucleotide sequence was inserted in an expression vector through restriction enzyme sites, and the expression vector was transfected into a host cell. Finally, the amino acid sequence of re-ApxIV toxin was expressed by the host cell and purified. In this Example, a DNA sequence encoding the epitope fragments of ApxIV toxin and a DNA sequence encoding pC3d-p31 bioadjuvant were linked with a *Hind*III restriction enzyme site, and the amino acid sequence of re-ApxIV toxin obtained by the cloning is SEQ ID NO: 92. The obtained re-ApxIV toxin was purified by nickel affinity chromatography and ion exchange chromatography. Then the purified re-ApxI toxin was confirmed by sodium dodecyl sulfate polyacrylamide gel electrophoresis (SDS-PAGE) and western blot, and the results are shown in Figure 1 (lane 4) and Figure 2 (lane 5), respectively. The results indicate that the re-ApxIV toxin has the expected molecular weight of about 50kDa.

**Example 5**

**Extraction of Apx Toxin**

1. Cultivation of *A. pleuropneumoniae*

Serotype 1 of *A. pleuropneumoniae* which produces ApxI, ApxII, and ApxIV (field isolated, Taiwan) and serotype 2 of *A. pleuropneumoniae* which produces ApxII, ApxIII, and ApxIV (field isolated, Taiwan) were separately cultivated in brain heart infusion (BHI) liquid medium containing 0.01% (v/v) $\beta$-Nicotinamide adenine dinucleotide ($\beta$-NAD) and 10% (v/v) horse serum (BD Biosciences, USA) overnight at 37°C, 5% $CO_2$.

2. Preparation of Apx Toxin

The serotypes 1 and 2 of *A. pleuropneumoniae* obtained above were broken by sonication (SONOPULS, Bandelin, Germany), and Apx toxin was collected by centrifugation (KUBOTA, Japan) followed by filtration of the supernatant with a 0.22 $\mu$m filter (Millipore, USA). The filtered crude extracted ApxI~IV toxin was stored at -80°C for further use.

3. Preparation of Inactivated *A. pleuropneumoniae*

Serotypes 1, 2, and 5 of *A. pleuropneumoniae* having the ability to produce toxin (field isolated, Taiwan) were separately cultivated in brain heart infusion (BHI) liquid medium containing 0.01% (v/v) $\beta$-Nicotinamide adenine dinucleotide ($\beta$-NAD) and 10% (v/v) horse serum (BD Biosciences, USA) overnight at 37°C, 5% $CO_2$. Then, the bacteria were inactivated with formaldehyde.

**Example 6**

**Preparation of Vaccine Against Porcine Pleuropneumonia**

1. Preparation of Subunit Vaccine of Recombinant Apx Toxin (re-ApxI, re-ApxII, re-ApxIII, and re-ApxIV)

Each of the re-ApxI toxin obtained in Example 1 (SEQ ID NO: 8)(with a final concentration of 500 $\mu$g/ml), re-ApxII toxin obtained in Example 2 (SEQ ID NO: 16)(with a final concentration of 500 $\mu$g/ml), re-ApxIII toxin obtained in Example 3 (SEQ ID NO: 21)(with a final concentration of 500 $\mu$g/ml), and re-ApxIV toxin obtained in Example 4 (SEQ ID NO: 92)(with a final concentration of 500 $\mu$g/ml) was separately mixed with aluminum hydroxide gel (with a final concentration of 30% (v/v)) in phosphate buffered solution (PBS). The aluminum hydroxide gel was the adjuvant of each of the subunit vaccine of recombinant Apx toxin.

2. Preparation of Multivalent *A. pleuropneumoniae* Bacterin (App 1, 2, 5)

The inactivated serotypes 1, 2, and 5 of *A. pleuropneumoniae* obtained in Example 5 (with a final concentration of 1 x $10^9$ cfu/ml for each serotype) were mixed in phosphate buffered solution (PBS). Aluminum hydroxide gel was added to a final concentration of 30% (v/v) in the mixture as an adjuvant of the multivalent *A. pleuropneumoniae* bacterin (App 1, 2, 5).

3. Preparation of Multivalent Vaccine Containing A. *pleuropneumoniae* Bacterin and Recombinant Apx Toxin (App 1, 2, 5 + re-ApxI~III)

The re-ApxI toxin obtained in Example 1 (SEQ ID NO: 8)(with a final concentration of 20 $\mu$g/ml), re-ApxII toxin obtained in Example 2 (SEQ ID NO: 16)(with a final concentration of 20 $\mu$g/ml), re-ApxIII toxin obtained in Example 3 (SEQ ID NO: 21)(with a final concentration of 20 $\mu$g/ml), and the inactivated serotypes 1, 2, and 5 of *A. pleuropneumoniae* obtained in Example 5 (with a final concentration of 1 x $10^9$ cfu/ml for each serotype) were mixed in phosphate buffered solution (PBS). Aluminum hydroxide gel was added to a final concentration of 30% (v/v) in the mixture as an adjuvant of the multivalent vaccine containing *A. pleuropneumoniae* bacterin and recombinant Apx Toxin against porcine pleuropneumonia (App 1, 2, 5 + re-ApxI~III).

**Example 7**

**Efficacy Test of Subunit Vaccine of Recombinant Apx Toxin**

1. Vaccination and Toxin Challenge in Mouse Model

Sixty (60) three-to-four-week-old healthy ICR mice (The National Laboratory Animal Center, Taiwan) were randomly divided into 6 groups (n = 10 for each group), in which Group 1 was negative control, and Groups 2 to 6 were vaccination groups. All of the mice were negative for anti-*A. pleuropneumoniae* antibodies. Each mouse was injected intraperitoneally (i.p.) with 0.2 ml of the following substance, respectively:

Group 1: PBS solution with 30% (v/v) of aluminum hydroxide gel (PBS group);
Group 2: Subunit vaccine containing the re-ApxI toxin obtained in Example 1 (SEQ ID NO: 8) (re-ApxI group);
Group 3: Subunit vaccine containing the re-ApxII toxin obtained in Example 2 (SEQ ID NO: 16) (re-ApxII group);

Group 4: Subunit vaccine containing the re-ApxIII toxin obtained in Example 3 (SEQ ID NO: 21) (re-ApxIII group);
Group 5: Subunit vaccine containing the re-ApxIV toxin obtained in Example 4 (SEQ ID NO: 92) (re-ApxIV group); and
Group 6: The multivalent *A. pleuropneumoniae* bacterin obtained in Example 6 (App 1, 2, 5 group).

Fourteen (14) days after primary immunization, each mouse was boosted with the same dose of the same substance respectively. Ten (10) days after booster immunization, each mouse was challenged by injection with 0.1 ml of the crude extracted ApxI~IV toxin obtained in Example 5, with a 90% lethal dose ($LD_{90}$)(extraction of $6.5 \times 10^9$ cfu/ml of Serotype 1 and $9.65 \times 10^{10}$ cfu/ml of Serotype 2 of *A. pleuropneumoniae*). Mice were observed for 10 days, and mortality of each group was recorded.

2. Statistical Analyses

Survival rate of each group was analyzed by Kaplan-Meier Survival Analysis: Log-Rank test. A statistically significant difference exists when the p-value is less than 0.05 ($p<0.05$).

3. Results

The results of toxin challenge are shown in Table 1. The results indicate that, comparing to negative control (Group 1, 0% survival rate), subunit vaccine containing either re-ApxI, re-ApxII, re-ApxIII, or re-ApxIV toxin (that is Group 2, 3, 4, or 5) induces a protective response against challenge with ApxI to ApxIV toxin in mice, with an increased survival rate having statistically significant difference.

Table 1 Survival Rate of Mice after being Challenged with ApxI to ApxIV Toxin

| Group | Number of Mice | Survival Rate |
| --- | --- | --- |
| Group 1 (PBS group) | 10 | 0% |
| Group 2 (re-ApxI group) | 10 | 50%* |
| Group 3 (re-ApxII group) | 10 | 50%* |
| Group 4 (re-ApxIII group) | 10 | 50%** |
| Group 5 (re-ApxIV group) | 10 | 30%* |
| Group 6 (App 1, 2, 5 group) | 10 | 20% |
| * indicates a statistically significant difference exists comparing to the negative group (PBS group) ($p<0.05$). ** indicates a statistically significant difference exists comparing to the negative group (PBS group) ($p<0.01$). | | |

**Example 8**

**Protection Efficacy of the Multivalent Vaccine Containing *A. pleuropneumoniae* Bacterin and Recombinant Apx Toxinx Against Serotype 2 of *A. pleuropneumoniae* (App 2)**

1. Vaccination and Challenge with Serotype 2 of *A. pleuropneumoniae* (App 2) in Mouse Model

Forty (40) three-to-four-week-old healthy ICR mice (The National Laboratory Animal Center, Taiwan) were randomly divided into 4 groups (n = 10 for each group), in which Group 1 was negative control, and Groups 2 to 4 were vaccination groups. All of the mice were negative for anti-*A. pleuropneumoniae* antibodies. Each mouse was injected intraperitoneally (i.p.) with 0.2 ml of the following substance, respectively:

Group 1: PBS solution with 30% (v/v) of aluminum hydroxide gel (PBS group);
Group 2: The multivalent *A. pleuropneumoniae* bacterin obtained in Example 6 (App 1, 2, 5 group);
Group 3: The multivalent vaccine containing *A. pleuropneumoniae* bacterin and recombinant Apx toxin obtained in Example 6 (App 1, 2, 5 + re-ApxI~III group); and
Group 4: A commercial *A. pleuropneumoniae* bacterin containing serotypes 1, 2, 3, 4, 5, and 7 (App 1, 2, 3, 4, 5, 7 group).

Fourteen (14) days after primary immunization, each mouse was boosted with the same dose of the same substance respectively. Ten (10) days after booster immunization, each mouse was challenged by injection with 0.1 ml of a 90% lethal dose (LD$_{90}$)(7.5x10$^8$ cfu/ml) of serotype 2 of *A. pleuropneumoniae* (App 2). Mice were observed for 10 days, and mortality of each group was recorded.

2. Statistical Analyses are the same as those described in Example 7.

3. Results

The results of challenge are shown in Table 2. The results indicate that, comparing to negative control (Group 1, 20% survival rate), each and every vaccination group (Groups 2, 3, and 4) induces a protective response against challenge with serotype 2 of *A. pleuropneumoniae* (App 2) in mice, with an increased survival rate having statistically significant difference. In particular, the multivalent vaccine containing *A. pleuropneumoniae* bacterin and the recombinant Apx toxin of the present invention (App 1, 2, 5 + re-ApxI~III group) has the best protective response with highest survival rate.

Table 2 Survival Rate of Mice after being Challenged with Serotype 2 of A. *pleuropneumoniae* (App 2)

| Group | Number of Mice | Survival Rate |
|---|---|---|
| Group 1 (PBS group) | 10 | 20% |
| Group 2 (App 1, 2, 5 group) | 10 | 70%** |
| Group 3 (App 1, 2, 5 + re-ApxI~III group) | 10 | 80%** |
| Group 4 (Commercial App 1, 2, 3, 4, 5, 7 group) | 10 | 60%* |
| * indicates a statistically significant difference exists comparing to the negative group (PBS group) ($p<0.05$). ** indicates a statistically significant difference exists comparing to the negative group (PBS group) ($p<0.01$). | | |

**Example 9**

**Protection Efficacy of the Multivalent Vaccine Containing *A. pleuropneumoniae* Bacterin and Recombinant Apx Toxinx Against Serotype 5 of *A. pleuropneumoniae* (App 5)**

1. Vaccination and Challenge with Serotype 5 of *A. pleuropneumoniae* (App 5) in Mouse Model

Fifty (50) three-to-four-week-old healthy ICR mice (The National Laboratory Animal Center, Taiwan) were randomly divided into 4 groups, in which Group 1 was negative control, and Groups 2 to 4 were vaccination groups. All of the mice were negative for anti-*A. pleuropneumoniae* antibodies. Each mouse was injected intraperitoneally (i.p.) with 0.2 ml of the following substance, respectively:

Group 1: PBS solution with 30% (v/v) of aluminum hydroxide gel (PBS group) (n = 15);
Group 2: The multivalent *A. pleuropneumoniae* bacterin obtained in Example 6 (App 1, 2, 5 group) (n = 15);
Group 3: The multivalent vaccine containing *A. pleuropneumoniae* bacterin and recombinant Apx toxin obtained in Example 6 (App 1, 2, 5 + re-ApxI~III group) (n = 10); and
Group 4: A commercial *A. pleuropneumoniae* bacterin containing serotypes 1, 2, 3, 4, 5, and 7 (App 1, 2, 3, 4, 5, 7 group) (n = 10).

Fourteen (14) days after primary immunization, each mouse was boosted with the same dose of the same substance respectively. Serum samples were collected 24 hours prior to primary immunization and 10 days after booster immunization. All the serum samples were tested by ELISA. Ten (10) days after booster immunization and blood sampling, each mouse was challenged by injection with 0.1 ml of a 90% lethal dose (LD$_{90}$)(7.32x10$^8$ cfu/ml) of serotype 5 of *A. pleuropneumoniae* (App 5). Mice were observed for 10 days, and mortality of each group was recorded.

2. Enzyme-linked immunosorbent assay (ELISA) of toxin antibodies

Recombinant ApxII toxin (re-ApxII, SEQ ID NO: 16) was coated on 96-well ELISA plates as antigen for 16 hours at 4°C. Uncoated antigen was removed, and the ELISA plates were washed 3 times with wash buffer (0.9% NaCl and 0.1% Tween 20) and air-dried. To block the ELISA plates, blocking solution (wash buffer containing 1% BSA) was added to the ELISA plates, and then the ELISA plates were incubated for an hour at room temperature. After that, the ELISA plates were washed with wash buffer. Mouse serum samples were diluted with PBS and added to the wells of the ELISA plates, and the plates were incubated for an hour at room temperature. After incubation, the serum samples were removed, and the plates were washed with PBS. Secondary antibody conjugated to horseradish peroxidase (HRP)(goat anti-mouse antibody conjugated HRP, Gene Tex Inc., USA) was diluted 5000-fold with blocking solution and then added to the wells (100 μl/well). After incubating for an hour at room temperature, the secondary antibody was removed, and the plates were washed with wash buffer. For visualization of results, 100 μl of 3, 3', 5, 5'-tetramethylbenzidine (TMB, KPL Inc., USA) was added to each well, and the plates were incubated for 10 minutes in dark. Optical density at 650nm ($OD_{650\,nm}$) was read with an ELISA plate reader (SpectraMax® M2/M2 ELISA Reader, Molecular Devices, LLC., USA).

3. Statistical Analyses

Survival rate of each group was analyzed by Kaplan-Meier Survival Analysis: Log-Rank test. A statistically significant difference exists when the p-value is less than 0.05 ($p<0.05$). Results of ELISA were analyzed by Student Newman-Keuls Method. A statistically significant difference exists when the p-value is less than 0.05 ($p<0.05$).

4. Results

The results of challenge are shown in Table 3. The results indicate that, comparing to negative control (Group 1, 6.7% survival rate), each and every vaccination group (Groups 2, 3, and 4) induces a protective response against challenge with serotype 5 of *A. pleuropneumoniae* (App 5) in mice, with an increased survival rate having statistically significant difference. In particular, the multivalent vaccine containing *A. pleuropneumoniae* bacterin and the recombinant Apx toxin of the present invention (App 1, 2, 5 + re-ApxI~III group) has the best protective response with highest survival rate.

Table 3 Survival Rate of Mice after being Challenged with Serotype 5 of A. *pleuropneumoniae* (App 5)

| Group | Number of Mice | Survival Rate |
|---|---|---|
| Group 1 (PBS group) | 15 | 6.7% |
| Group 2 (App 1, 2, 5 group) | 15 | 26.7%* |
| Group 3 (App 1, 2, 5 + re-ApxI~III group) | 10 | 70%** |
| Group 4 (Commercial App 1, 2, 3, 4, 5, 7 group) | 10 | 60%* |
| * indicates a statistically significant difference exists comparing to the negative group (PBS group) ($p<0.05$). ** indicates a statistically significant difference exists comparing to the negative group (PBS group) ($p<0.01$). | | |

ELISA results are shown in Figure 3. Ten (10) days after booster immunization, the multivalent vaccine containing *A. pleuropneumoniae* bacterin and the recombinant Apx toxin of the present invention (Group 3, i.e. App 1, 2, 5 + re-ApxI~III group) induced significantly higher level of serum antibodies against Apx toxin in mice than negative control (Group 1, i.e. PBS group, $p<0.01$), the multivalent *A. pleuropneumoniae* bacterin (Group 2, i.e. App 1, 2, 5 group, $p<0.01$), and the commercial *A. pleuropneumoniae* bacterin containing serotypes 1, 2, 3, 4, 5, and 7 (Group 4, i.e. App 1, 2, 3, 4, 5, 7 group, $p<0.01$) did. Therefore, the results indicated that the multivalent vaccine containing *A. pleuropneumoniae* bacterin and the recombinant Apx toxin of the present invention is able to effectively induce immune responses against Apx toxin in mice and has immunogenicity significantly better than the commercial *A. pleuropneumoniae* vaccine.

**Example 10**

**Preparation of Anti-ApxI to ApxIV Polyclonal Antibodies**

Each of the re-ApxI toxin obtained in Example 1 (SEQ ID NO: 8), re-ApxII toxin obtained in Example 2 (SEQ ID NO: 16), re-ApxIII toxin obtained in Example 3 (SEQ ID NO: 21), and re-ApxIV toxin obtained in Example 4 (SEQ ID NO: 92) was separately mixed with Freund's complete adjuvant (FCA, Sigma, USA). Each of the mixture was primarily inoculated intraperitoneally into Balb/c mice (0.2 ml/mouse), and a second immunization was performed after 3 weeks. After one week, serum of the inoculated mice was collected as anti-Apx1/2/3/4 polyclonal antibodies. The polyclonal antibodies are used for western blot.
The foregoing description of the exemplary embodiments of the invention has been presented only for the purposes of illustration and description and is not intended to be exhaustive or to limit the invention to the precise forms disclosed. Many modifications and variations are possible in light of the above teaching.

**Claims**

1. A recombinant *Actinobacillus pleuropneumoniae* toxin (re-Apx) represented by formula (I):

$$(A)_m\text{-}(C3d\ fragment)_n \qquad\qquad (I)$$

wherein

each A is an individual epitope of an *Actinobacillus pleuropneumoniae* toxin (Apx);
each C3d fragment is an individual unit of the amino acid sequence of complement C3d and is independently selected from the group consisting of SEQ ID NOs: 22, 23, 24, and 25;
m is an integer from 1 to about 30; and
n is an integer from 0 to about 10.

2. The recombinant *Actinobacillus pleuropneumoniae* toxin (re-Apx) of claim 1, wherein each A is linked to another A with a linker, and each linker is independently selected from the group consisting of Gly-Gly, Gly-Ser, and SEQ ID NOs: 26, 27, 28, 29, 30, 31, 32, 33, 34, 35, and 36.

3. The recombinant *Actinobacillus pleuropneumoniae* toxin (re-Apx) of claim 1, wherein each C3d fragment is linked to another C3d fragment with a linker, and each linker is independently selected from the group consisting of Gly-Gly, Gly-Ser, and SEQ ID NOs: 26, 27, 28, 29, 30, 31, 32, 33, 34, 35, and 36.

4. The recombinant *Actinobacillus pleuropneumoniae* toxin (re-Apx) of claim 1, wherein the A next to a C3d fragment is linked to the C3d fragment with a linker, and the linker is selected from the group consisting of Gly-Gly, Gly-Ser, and SEQ ID NOs: 26, 27, 28, 29, 30, 31, 32, 33, 34, 35, and 36.

5. The recombinant *Actinobacillus pleuropneumoniae* toxin (re-Apx) of claim 1, wherein the *Actinobacillus pleuropneumoniae* toxin (Apx) is ApxI toxin, the recombinant *Actinobacillus pleuropneumoniae* toxin is re-ApxI toxin, and each A is independently selected from the group consisting of the amino acid sequences of SEQ ID NOs: 2, 3, 4, 5, 6, 37, 39, 40, 41, 42, 43, 44, 45, 46, 47, 48, 49, 50, and 51.

6. The recombinant *Actinobacillus pleuropneumoniae* toxin (re-Apx) of claim 5, wherein the re-ApxI toxin comprises the amino acid sequences of SEQ ID NOs: 7 or 8.

7. The recombinant *Actinobacillus pleuropneumoniae* toxin (re-Apx) of claim 1, wherein the *Actinobacillus pleuropneumoniae* toxin (Apx) is ApxII toxin, the recombinant *Actinobacillus pleuropneumoniae* toxin is re-ApxII toxin, and each A is independently selected from the group consisting of the amino acid sequences of SEQ ID NOs: 10, 11, 12, 13, 14, 52, 53, 54, 55, 56, 57, 58, 59, 60, 61, 62, 63, 64, 65, 67, and 68.

8. The recombinant *Actinobacillus pleuropneumoniae* toxin (re-Apx) of claim 7, wherein the re-ApxII toxin comprises the amino acid sequences of SEQ ID NOs: 15 or 16.

9. The recombinant *Actinobacillus pleuropneumoniae* toxin (re-Apx) of claim 1, wherein the *Actinobacillus pleuropneumoniae* toxin (Apx) is ApxIII toxin, the recombinant *Actinobacillus pleuropneumoniae* toxin is re-ApxIII toxin, and each A is independently selected from the group consisting of the amino acid sequences of SEQ ID NOs: 18, 19, 69, 70, 71, 72, 73, 74, 75, 76, 77, 78, 79, 80, 81, 82, 83, 84, 85, 86, 87, and 88.

10. The recombinant *Actinobacillus pleuropneumoniae* toxin (re-Apx) of claim 9, wherein the re-ApxIII toxin comprises the amino acid sequences of SEQ ID NOs: 20 or 21.

11. The recombinant *Actinobacillus pleuropneumoniae* toxin (re-Apx) of claim 1, wherein the *Actinobacillus pleuropneumoniae* toxin (Apx) is ApxIV toxin, the recombinant *Actinobacillus pleuropneumoniae* toxin is re-ApxIV toxin, and each A is independently selected from the group consisting of the amino acid sequences of SEQ ID NOs: 66, 89, 90, 93, 94, 95, 96, 97, 98, 99, 100, 101, 102, 103, 104, 105, 106, 107, 108, 109, 110, 111, 112, 113, 114, 115, 116, and 117.

12. The recombinant *Actinobacillus pleuropneumoniae* toxin (re-Apx) of claim 11, wherein the re-ApxIV toxin comprises the amino acid sequences of SEQ ID NOs: 91 or 92.

13. A polynucleotide comprising a nucleotide sequence encoding the recombinant *Actinobacillus pleuropneumoniae* toxin (re-Apx) of claim 1.

14. An immunogenic composition against porcine pleuropneumonia, comprising the recombinant *Actinobacillus pleuropneumoniae* toxin (re-Apx) of claim 1 and a pharmaceutically acceptable vehicle.

15. The immunogenic composition against porcine pleuropneumonia of claim 14, wherein the recombinant *Actinobacillus pleuropneumoniae* toxin (re-Apx) is selected from at least one of the group consisting of the re-ApxI toxin of claim 5, the re-ApxII toxin of claim 7, the re-ApxIII toxin of claim 9, and the re-ApxIV toxin of claim 11.

16. The immunogenic composition against porcine pleuropneumonia of claim 14, further comprising at least one serotype of *A. pleuropneumoniae.*

17. The immunogenic composition against porcine pleuropneumonia of claim 14, further comprising at least one pathogen antigen selected from the group consisting of antigen of porcine circovirus type 2 (PCV2), antigen of Swine influenza virus (SIV), antigen of porcine reproductive and respiratory syndrome virus (PRRSV), antigen of mycoplasma, antigen of porcine parvovirus (PPV), antigen of erysipelas, antigen of *Bordetella bronchiseptica,* antigen of *Pasteurella multocida,* and antigen of pseudorabies (Aujeszky's disease) virus.

18. A method of protecting an animal against porcine pleuropneumonia, comprising administering the immunogenic composition of claim 14 to the animal to increase immunity against porcine pleuropneumonia in the animal.

19. An anti-*Actinobacillus pleuropneumoniae* toxin (Apx) antibody derived from the recombinant *Actinobacillus pleuropneumoniae* toxin (re-Apx) of claim 1.

20. The anti-*Actinobacillus pleuropneumoniae* toxin (Apx) antibody of claim 19, wherein the antibody comprises at least one of a monoclonal antibody, a polyclonal antibody, and a genetically engineered antibody.

21. A test kit for porcine pleuropneumonia, comprising a detecting unit which is at least one of the recombinant *Actinobacillus pleuropneumoniae* toxin (re-Apx) of claim 1 and an anti-*Actinobacillus pleuropneumoniae* toxin (Apx) antibody derived from the recombinant *Actinobacillus pleuropneumoniae* toxin (re-Apx) of claim 1.

22. The test kit of claim 21, wherein the antibody comprises at least one of a monoclonal antibody, a polyclonal antibody, and a genetically engineered antibody.

Figure 1

Figure 2

Figure 3

EP 3 450 461 A1

# INTERNATIONAL SEARCH REPORT

International application No.

**PCT/CN2016/080469**

## A. CLASSIFICATION OF SUBJECT MATTER

C07K 19/00 (2006.01) i; C07K 14/36 (2006.01) i; C12N 15/62 (2006.01) i; C07K 16/12 (2006.01) i; A61K 39/02 (2006.01) i; A61P31/04 (2006.01) i; G01N 33/53 (2006.01) i

According to International Patent Classification (IPC) or to both national classification and IPC

## B. FIELDS SEARCHED

Minimum documentation searched (classification system followed by classification symbols)
C07K; C12N; A61K; A61P; G01N

Documentation searched other than minimum documentation to the extent that such documents are included in the fields searched

Electronic data base consulted during the international search (name of data base and, where practicable, search terms used)

CNABS; CPRSABS; CNTXT; DWPI; SIPOABS; EPTXT; WOTXT; USTXT; JPTXT; CNKI; PUBMED AND KEYWORDS: complement split fragment, Apx, actinobacillus pleuropneumoniae, porcine pleuropneumonia, App, recombinant protein, fusion protein, toxin, antigenic determinant, epitope, C3d; CHINA PATENT BIOLOGICAL SEQUENCE SEARCH SYSTEM; Genbank; EMBL AND SEARCHED SEQUENCES: SEQ ID NOs: 1-117

## C. DOCUMENTS CONSIDERED TO BE RELEVANT

| Category* | Citation of document, with indication, where appropriate, of the relevant passages | Relevant to claim No. |
|---|---|---|
| X | CN 104059134 A (SBC VIRBAC BIOTECHNOLOGY CO., LTD.), 24 September 2014 (24.09.2014), see the whole document, particular claims 12, 14-17 and 15-28, description, embodiment 1, and sequence listing | 1-22 |
| A | CN 1511844 A (ZHANG, Gannan et al.), 14 July 2004 (14.07.2004), see the whole document | 1-22 |
| A | CN 101691369 A (TIANJIN AGRICULTURAL UNIVERSITY), 07 April 2010 (07.04.2010), see the whole document | 1-22 |
| A | EP 2769734 A1 (INTERVET INT BV), 27 August 2014 (27.08.2014), see the whole document | 1-22 |

☐ Further documents are listed in the continuation of Box C.　　☒ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 27 July 2016 (27.07.2016) | **03 August 2016 (03.08.2016)** |

| Name and mailing address of the ISA/CN: State Intellectual Property Office of the P. R. China No. 6, Xitucheng Road, Jimenqiao Haidian District, Beijing 100088, China Facsimile No.: (86-10) 62019451 | Authorized officer **WANG, Ying** Telephone No.: (86-10) **62089434** |

Form PCT/ISA/210 (second sheet) (July 2009)

## INTERNATIONAL SEARCH REPORT

International application No.

**PCT/CN2015/000624**

**Box No. II    Observations where certain claims were found unsearchable (Continuation of item 2 of first sheet)**

This international search report has not been established in respect of certain claims under Article 17(2)(a) for the following reasons:

1. ☒    Claims Nos.: 18
   because they relate to subject matter not required to be searched by this Authority, namely:

[1]    PCT Rule 39.1(iv) —a method by surgery or therapy for the treatment of a human or animal body. The subject matter of the claim mentioned above that is reasonably expected is: the use of the immune combination as claimed in claim 14 in the preparation of drugs for treating an actinobacillus pleuropneumoniae.

2. ☐    Claims Nos.:
   because they relate to parts of the international application that do not comply with the prescribed requirements to such an extent that no meaningful international search can be carried out, specifically:

3. ☐    Claims Nos.:
   because they are dependent claims and are not drafted in accordance with the second and third sentences of Rule 6.4(a).

Form PCT/ISA/210 (continuation of first sheet (2)) (July 2009)

**INTERNATIONAL SEARCH REPORT**
Information on patent family members

| International application No. |
| --- |
| **PCT/CN2016/080469** |

| Patent Documents referred in the Report | Publication Date | Patent Family | Publication Date |
| --- | --- | --- | --- |
| CN 104059134 A | 24 September 2014 | WO 2014146603 A1 | 25 September 2014 |
| | | EP 2982683 A1 | 10 February 2016 |
| | | TW 201437224 A | 01 October 2014 |
| CN 1511844 A | 14 July 2004 | None | |
| CN 101691396 A | 07 April 2010 | None | |
| EP 2769734 A1 | 27 August 2014 | EP 2461823 A1 | 13 June 2012 |
| | | JP 2013501028 A | 10 January 2013 |
| | | KR 20120054037 A | 29 May 2012 |
| | | US 2012128712 A1 | 24 May 2012 |
| | | RU 2554812 C2 | 27 June 2015 |
| | | RU 2012108227 A | 20 September 2013 |
| | | JP 5847713 B2 | 27 January 2016 |
| | | EP 2461823 B1 | 04 June 2014 |
| | | DK 2461823 T3 | 21 July 2014 |
| | | CN 102481350 A | 30 May 2012 |
| | | WO 2011015614 A1 | 10 February 2011 |
| | | ES 2492675 T3 | 10 September 2014 |
| | | BR 112012002583 A2 | 22 March 2016 |
| | | TW 201109439 A | 16 March 2011 |
| | | MX 2012001577 A | 26 March 2012 |

Form PCT/ISA/210 (patent family annex) (July 2009)